# EUROPEAN PATENT APPLICATION

(11) **EP 2 428 584 A1**
(43) Date of publication of application: **14.03.2012**
(21) Application number: 10758307.2
(22) Date of filing: 05.04.2010
(51) Int. Cl.: C12Q 1/68, C12N 15/09, G01N 33/50

(54) **METHOD FOR DETECTION OF COLORECTAL TUMOR**

(30) Priority: 03.04.2009 JP 2009091487; 18.11.2009 JP 2009262791
(71) Applicant: A & T Corporation, Fujisawa-shi Kanagawa 252-0816 (JP)
(72) Inventor: HINODA, Yuji, Ube-shi Yamaguchi 755-8505 (JP); SUEHIRO, Yutaka, Ube-shi Yamaguchi 755-8505 (JP); UENO, Koji, Ube-shi Yamaguchi 755-8505 (JP); OKADA, Toshiyuki, Ube-shi Yamaguchi 755-8505 (JP)
(74) Representative: Griffin, Philippa Jane
(86) International application number: PCT/JP2010/002490
(87) International publication number: WO 2010/113529

(57) **Abstract**

Disclosed is a method for determining the presence or absence of a colorectal tumor, specifically colorectal cancer or colorectal adenoma, with high sensitivity and high specificity by employing the methylation of DNA as a measure. Also disclosed is a kit for carrying out the method. Specifically, measurement is made on the degree of methylation of one or more CpG sequences contained in the region lying between positions -477 to -747, more preferably a CGCG sequence contained in the region lying between positions -688 to -691, in TWIST1 gene (Homo sapiens twist homolog 1; Drosophila gene) located on the genome sequence of a test cell.

<110> Yamaguchi University

<120> Method for detection of colorectal tumor

<130> P37254EP-PCT

<140> EP 10758307.2
>141> 2010-04-05

<150> JP 2009-091487
<151> 2009-04-03

<150> JP 2009-262791
<151> 2009-11-18

<160> 5

<210> 1
<211> 444
<212> DNA
<213> Homo sapiens

<400> 1

TGTGTAGAAG CTGTTGCCAT TGCTGCTGTC ACAGCCACTC CGGATGGGGC TGCCACCGCG 60

GCCAGGACAG TCTCCTCCGA CCGCTTCCTG GGCTGCGCTA GGGTTCGGGG GCGCTGCCCG 120

CACGCTCCGG CGGGGAAGGA AATCGCCCCG CGCCCGCCGG AGGAAGGCGA CGGGGAGGGA 180

AGGGGGAGGG CGGCTAGGAG GCGGGTGGAG GGGCCGGCCG CCCGGGCCAG GTCGTTTTTG 240

AATGGTTTGG GAGGACGAAT TGTTAGACCC CGAGGAAGGG AGGTGGGACG GGGGAGGGGG 300

ACTGGAAAGC GGAAACTTTC CTATAAAACT TCGAAAAGTC CCTCCTCCTC ACGTCAGGCC 360

AATGACACTG CTGCCCCCAA ACTTTCCGCC TGCACGGAGG TATAAGAGCC TCCAAGTCTG 420

CAGCTCTCGC CCAACTCCCA GACA 444

<210> 2
<211> 22
<212> DNA
<213> Artificial sequence

<400> 1

TGTGTAGAAG TTGTTGTTAT TG 22

<210> 3
<211> 20
<212> DNA
<213> Artificial sequence

<400> 1

CRAAAAAAAC TATCCTAACC                                                    20

<210> 4
<211> 21
<212> DNA
<213> Artificial sequence

<400> 1

TGTGTAGAAGTTGTTGTTATT                                                    21

<210> 5
<211> 19
<212> DNA
<213> Artificial sequence

<400> 1

CRAAAAAAAC TATCCTAAC                                                     19

## Description

### Technical Field

The present invention relates to a method for determining the presence or absence of colorectal tumor, and more specifically to a method for determining colorectal cancer or colorectal adenoma and a kit for determining colorectal tumor, wherein the degree of methylation of CpG sequence is measured in the region between positions -477 and -747 of Homo sapiens twist homolog 1 (Drosophila) gene (hereinafter described as TWIST1) in the genome sequence of a test cell.

### Background Art

Colorectal tumor occurring in the cecum, colon and rectum is a concept including colorectal adenoma as a benign tumor and colorectal cancer which is of high malignancy and sometimes has metastatic properties. Among them, colorectal cancer is the current second most common cancer in Japan and is known to be mostly caused by the mutation of mucosal epithelial cells of the digestive tract lining. The main cause of the canceration of the mucosal epithelial cells is considered to be mutation on the genomic DNA controlling cell proliferation, and other environmental factors (risk factors), heredity, viral infection, and the like are known as factors influencing the canceration thereof.

Like many cancers, it is also more important than anything else to early detect the colorectal cancer for the treatment thereof, and many test methods have hitherto been developed. The major test methods include a stool test for occult blood, a blood test, digital rectal palpation, and a diagnosis using a colorectal endoscope; however, each of the test methods has a problem with sensitivity particularly in early cancer: the stool test for occult blood has a problem with detection sensitivity in early cancer; a problem of the blood test (a test of a tumor marker contained in the blood) also is to show positivity only for advanced cancer; a problem of the digital rectal palpation is to only enable diagnosis within reach of a finger; or a problem of the diagnosis using a colorectal endoscope is also to have reduced detection sensitivity in early cancer less easy to identify visually.

By focusing attention on the occurrence of mutation in the genomic DNA of cells cancerated in the future in the early stage of cancer, methods for detecting cancer have been developed in which the mutation of DNA and its expression product RNA, especially the methylation of DNA, is used as a measure of canceration. Patent Document 1 discloses a human DNA sequence related to canceration. Also disclosed are a detection method for colorectal cancer using methylation in the sequence of vimentin gene as a measure (Patent Document 2), a detection method for cancer using the methylation of the SPARC gene as a measure (Patent Document 3), a detection method for cancer using the mRNA expression information of ceramide hydroxylase (Patent Document 4), a detection method for colorectal cancer using the mutation of APC, K-ras and p53 genes as a measure (Patent Document 5), a detection method for digestive system cancer using the methylation of APC and/or DCC genes as a measure (Patent Document 6), and the like. However, each of the methods has problems in terms of detection sensitivity, specificity, cost, and the like and is not clinically put to practical use at this moment.

Based on these present situations, there has been a need for development of a method for early and sensitively detecting digestive system tumor, especially colorectal tumor, specifically colorectal cancer or colorectal adenoma as a function of the degree of progression and with high specificity by using less gene information, more specifically methylation in the sequence of one gene, as a measure.

On the one hand, the TWIST1 gene is a gene isolated as a human homolog of the gene of a transcriptional regulatory factor, TWIST, responsible for the morphogenesis of *Drosophila melanogaster* and also called H-TWIST or a TWIST homolog of Drosophila. The TWIST1 gene is essentially a gene of a basic helix-loop-helix (bHLH)-type transcriptional regulatory factor and is generally considered to be involved in the lineage and differentiation of cells during the development thereof. A mutation occurring in the TWIST1 gene per se (coding region) is known to cause Seathre-Chotzen syndrome and has recently been suggested to be involved in the taxol resistance acquisition of nasopharyngeal cancer cells. However, the fact has not previously been known that the methylation of TWIST1 gene, particularly methylation present in the regulatory region or neighboring sequence, strongly correlates with the canceration of colorectal cells.

On the other hand, in studies in breast cancer patients, the methylated alleles of Cyclin D2, RAR-β and Twist detected from a patient's lactiferous duct-derived fluid containing cancer visualized with an endoscope were examined using methylation-specific PCR (MSP) (Non-Patent Document 1), and RASSF1A, HIN-1, RAR-β, Cyclin D2 and Twist known as cancer-related genes were examined in lobular carcinoma in situ (ILC) and invasive lobular carcinoma (ILC) by a methylation-specific PCR (MSP) method, which confirmed that 100% of ILC and 69% of LCIS had one or two or more hyper-methylated genes (Non-Patent Document 2). It is reported that in cancer patients, a Q-PCR method demonstrated increases in the expression of Slug and Twist and a decrease in the expression of Snail and methylation in the Twist promoter (Non-Patent Document 3) and that in human breast cancer, abnormal hypermethylation was shown in any one or more of Twist, cyclin D2, retinoic acid, RARβ and HIN-1 (Non-Patent Document 4). A method is reported for administering a methylation-regulating agent selected from a DNA methylation inhibitor, a demethylating agent, and an antagonist for DNA methyltransferase activity into the lactiferous duct of patients having abnormal epithelial cells of the lactiferous duct containing typical or malignant cells (Patent Document 7). However, for example, the methylation-sensitive PCR (MSP) method using primers each containing at least one CG dinucleotide, one of which needs to be located at the 3' terminal thereof, is excellent in the detection of methylated sequence but unsuitable for quantitative detection.

### Prior Art Documents

### Patent Documents

Patent Document 1: Japanese Unexamined Patent Application Publication (Translation of PCT Application) No. 2004-527245
Patent Document 2: Japanese Unexamined Patent Application Publication (Translation of PCT Application) No. 2007-502121
Patent Document 3: Japanese Unexamined Patent Application Publication (Translation of PCT Application) No. 2007-524393
Patent Document 4: Japanese Unexamined Patent Application Publication No. 2007-252272
Patent Document 5: Japanese Unexamined Patent Application Publication No. 2007-274926
Patent Document 6: Japanese Unexamined Patent Application Publication No. 2006-166732
Patent Document 7: Japanese Unexamined Patent Application Publication No. 2009-96808

### Non-patent Documents

Non-Patent Document 1: Evron E. et al. 2001. Detection of breast cancer cells in ductal lavage fluid by methylation-specific PCR. The Lancet 357:1335-1336
Non-Patent Document 2: Fackler M.J. et al. 2003. DNA methylation of RASSF1A, HIN-1, RAR-b, CYCLIN D2 and TWIST in in situ and invasive lobular breast carcinoma. Int.J.Cancer 107:970-975
Non-Patent Document 3: Tracey A. et al. 2005. Expression of the transcription factors snail, slug, and twist and their clinical significance in human breast cancer.
Non-Patent Document 4: Bae Y.K. et al. 2005. Gene promoter hypermethylation in tumors and plasma of breast cancer patients. Cancer Res Treat.37(4):233-240

### Summary of the Invention

### Object to be Solved by the Invention

With the foregoing current circumstances in view, an object of the present invention is to provide a method for detecting colorectal tumor with high sensitivity and with high specificity and a kit for carrying out the same.

### Means to Solve the Object

Based on the findings that the methylation of CpG islands of various genes in breast cancer cells was associated with breast cancer, the present inventors searched for methylated regions of various genes enabling the specific detection of colorectal tumor. As a result, it was found that highly methylated sites are also present in many genes of colorectal tumor cells. However, it was recognized that a method using this fact has a problem with detection specificity because, for example, SFRP gene was shown to have a high methylation frequency in colorectal cancer while also having a high methylation frequency of 68.0% in normal colorectal mucosa and similarly a plurality of other genes having a high methylation frequency in colorectal cancer cells tended to also have a high methylation frequency in normal colorectal mucosa. The relationship between cancer of other organs and methylated regions of various genes was also examined; however, it was confirmed that the correlation, for example, between the methylation in CpG of TWIST1 and stomach cancer could not be found. However, for further detailed studies, by focusing attention on highly methylated sites of the 5' upstream regulatory region of human TWIST1 in liver cancer cells, the rate of methylation has been compared between normal individuals and colorectal tumor and colorectal cancer patients, using normal colorectal mucosa and clinical specimens of colorectal cancer and colorectal adenoma. As a result, it has surprisingly been found that the methylation of the CGCG site at positions -688 to -691 in the human TWIST1 promoter region has a strong correlation with the presence or absence of colorectal tumor and with the degree of progression of the tumor. As a result of further studies based on the above findings, the present inventors have found that the methylation of the CpG site in the region between positions -477 and -747 in the human TWIST1 promoter region can be used as a marker for the detection and discrimination of colorectal tumor, thereby accomplishing the present invention.

Thus, the present invention relates to: (1) a method for determining a presence or absence of colorectal tumor, comprising measuring a degree of methylation of one or more CpG sequence(s) in the region between positions -477 and -747 of TWIST1 gene in the genome sequence of a test cell; (2) the determination method according to (1) above, wherein when it is determined that there is a colorectal tumor, the colorectal tumor is further determined to be cancer or adenoma; (3) the determination method according to (1) or (2) above, wherein the one or more CpG sequence(s) in the region between positions -477 and -747 of the TWIST1 gene is the CGCG sequence located at positions 57 to 60 in the nucleotide sequence represented by SEQ ID NO: 1; (4) the determination method according to (3) above, wherein the degree of methylation is measured by a COBRA (Combined Bisulfite Restriction Analysis) method and the CGCG sequence located at positions 57 to 60 in the nucleotide sequence represented by SEQ ID NO: 1 is amplified using a combination of 5'-TGTGTAGAAGTTGTTGTTATT-3' (SEQ ID NO: 4) and 5'-CRAAAAAAACTATCCTAAC-3' (SEQ ID NO: 5) as a primer set in an amplification step; and (5) the determination method according to any one of (1) to (4) above, wherein the test cell is a cell contained in feces, a cell contained in a colorectal wash, a cell contained in blood, or a cell contained in serum.

The present invention also relates to (6) a colorectal tumor determination kit for measuring a degree of methylation of one or more CpG sequences in a region between positions -477 and -747 of TWIST1 gene in the genome sequence of a test cell, comprising a reagent for extracting genomic DNA, a reagent for converting unmethylated cytosine to uracil, a primer set for amplifying one or more CpG sequences in the region between positions -477 and -747 of TWIST1 gene in the genomic DNA, and a reagent for analyzing a methylation pattern; and (7) the colorectal tumor determination kit according to (6) above, wherein the primer set is a primer pair consisting of the oligonucleotides represented by SEQ ID NO: 4 and SEQ ID NO: 5.

### Effect of the Invention

The method for detecting colorectal tumor according to the present invention can be used to discriminate between tumor such as colorectal cancer and non-tumor tissue with high sensitivity and with high specificity, and can also be used for the determination of the degree of progression of the tumor since the methylation level of the tumor increases depending on the progression thereof. In addition, a kit or the like for carrying out the method can be used to simply detect colorectal tumor.

### Brief Description of Drawings

[Figure 1] Figure 1 is a set of photographs showing the results of COBRA assay according to present invention. (A) shows electrophoretic images derived from normal colorectal mucosa and (B) shows electrophoretic images derived from colorectal cancer cells.
[Figure 2] Figure 2 is a drawing showing quantitative values of methylation in samples of clinical specimens (colorectal cancer).
[Figure 3] Figure 3 is a drawing showing quantitative values of methylation in samples of clinical specimens (normal colorectal mucosa).
[Figure 4] Figure 4 is a graph showing the comparison of the methylation level of TWIST1 gene in colorectal cancer cells and normal colorectal mucosa cells.
[Figure 5] Figure 5 is a graph showing an ROC curve and a cutoff value derived from the results in Figure 2 and Figure 3.
[Figure 6] Figure 6 is a drawing showing the results of ROC analysis between colorectal cancer and normal colorectal mucosa at cutoff values of 0.45 to 21.20%.
[Figure 7] Figure 7 is a drawing showing the results of ROC analysis between colorectal cancer and normal colorectal mucosa at cutoff values of 21.95 to 40.70%.
[Figure 8] Figure 8 is a drawing showing the results of ROC analysis between colorectal cancer and normal colorectal mucosa at cutoff values of 41.05 to 58.55%.
[Figure 9] Figure 9 is a drawing showing the results of ROC analysis between colorectal cancer and normal colorectal mucosa at cutoff values of 58.70 to 83.60%.
[Figure 10] Figure 10 is a drawing showing the results of ROC analysis between colorectal cancer and normal colorectal mucosa at cutoff values of 83.85 to 99.60%.
[Figure 11] Figure 11 is a graph in which the cutoff value derived from the ROC analysis was applied to the graph in Figure 4.
[Figure 12] Figure 12 is a drawing showing the methylation frequency of TWIST1 in colorectal cancer in comparison with other conventionally reported genes.
[Figure 13] Figure 13 is a set of photographs showing the results of COBRA assay using DNAs derived from normal colorectal mucosa, colorectal cancer and colorectal adenoma as templates.
[Figure 14] Figure 14 is a drawing showing quantitative values of methylation in samples of colorectal adenoma specimens.
[Figure 15] Figure 15 is a graph showing the methylation level of TWIST1 gene in colorectal adenoma in comparison with normal colorectal mucosa and colorectal cancer.
[Figure 16] Figure 16 is a graph showing an ROC curve and cutoff value of the methylation of TWIST1 gene in colorectal adenoma and normal colorectal mucosa.
[Figure 17] Figure 17 is a drawing showing the results of ROC analysis between colorectal adenoma and normal tissue at cutoff values of 0.05 to 8.650.
[Figure 18] Figure 18 is a drawing showing the results of ROC analysis between colorectal adenoma and normal tissue at cutoff values of 8.750 to 37.40.
[Figure 19] Figure 19 is a drawing showing the results of ROC analysis between colorectal adenoma and normal tissue at cutoff values of 38.30 to 99.05.
[Figure 20] Figure 20 is a graph in which the cutoff value derived from the ROC analysis was applied to the graph for normal colorectal mucosa and colorectal adenoma in Figure 15.
[Figure 21] Figure 21 is a drawing showing the results of ROC analysis between colorectal cancer and colorectal adenoma at cutoff values of 0.800 to 14.95.
[Figure 22] Figure 22 is a drawing showing the results of ROC analysis between colorectal cancer and colorectal adenoma at cutoff values of 15.25 to 29.85.
[Figure 23] Figure 23 is a drawing showing the results of ROC analysis between colorectal cancer and colorectal adenoma at cutoff values of 30.05 to 45.95.
[Figure 24] Figure 24 is a chart showing the analysis of distribution of CpG methylation in the region between positions -477 and -747 of TWIST1 gene in the colorectal cancer cell lines DLD-1, HT-29, HCT-116 and RKO, methylation-negative control (NL, derived from peripheral blood lymphocytes), and methylation-positive control (IVD, DNA treated with methylation transferase).
[Figure 25] Figure 25 is a graph showing the comparison of the methylation level of TWIST1 gene in stomach cancer cells and normal stomach mucosa.

### Mode of Carrying Out the Invention

The method for determining the presence or absence of colorectal tumor according to the present invention is not particularly limited provided that it is a method for measuring the degree (frequency, rate, or the like) of the methylation of one or more CpG sequences in the region between positions -477 and -747 of TWIST1 gene in the genome sequence of a test cell. The colorectal tumor is a concept including colorectal cancer or colorectal adenoma produced by the transformation of normal cells in a tissue. Here, the colorectal adenoma means dysplastic colorectal cells in a precancerous state or a non-metastatic benign tumor; the colorectal cancer means a metastatic malignant tumor; and the progression of colorectal tumor means the proliferation of colorectal adenoma in the normal colorectal tissue, the new conversion of precancerous colorectal adenoma to cancer, or the proliferation of cancer cells metastasized from a cancer part in non-cancerous colorectal tissues to newly form colorectal cancer tissues.

The measurement of the degree of methylation (frequency, rate, or the like) of CpG sequence specifically refers to the measurement of the presence, frequency, rate, or the like of the methylation of cytosine present in the form of CpG sequence in positions -477 to -747 in TWIST1 promoter region, particularly preferably the methylation of cytosine present in CGCG sequence in positions -688 to -691 (positions 57 to 60 in the base sequence described in SEQ ID NO: 1). Methods for measuring the degree of methylation can include a COBRA (Combined Bisulfite Restriction Analysis) method, a Methyl Light method, a Pyrosequencing method, a Bisulfite sequencing method, a MassArray method, a qAMP (Restriction and real-time PCR) method, an RT-LAMP method, and an ICAN method; among others, preferred examples thereof can include a COBRA (Combined Bisulfite Restriction Analysis) method.

The method for determining the presence or absence of colorectal tumor according to the present invention is a method comprising (1) a step a of extracting genomic DNA from a cell to be tested; (2) a step b of converting unmethylated cytosine in CpG sequence in the DNA obtained in the step a to a different base (preferably uracil); (3) a step c of amplifying the region between positions -477 and -747 of TWIST1 gene subjected to the conversion in the step b or the CpG site contained in the region by a PCR method using a set of primers; and (4) a step d of measuring the degree of methylation (analyzing the methylation pattern) in the amplification product obtained in the step c. In the step d, the rate of methylation can be calculated followed by applying a predetermined cutoff value thereto, resulting in the statement that colorectal cancer is considered present when it is high and that colorectal adenoma is considered present when it is low. In this respect, the step d may be a step of collecting data for analyzing the methylation pattern. More suitably, the method is a method in which the step of analysis in the step d comprises restriction enzyme (methylation-specific restriction enzyme) treatment enabling the discrimination between the methylation and the non-methylation of a target sequence.
The set of primers in the step c can be properly designed based on the sequence information of TWIST1 and properly produced using a suitable oligonucleotide synthesizer, and are not particularly limited in the position, length or the like thereof provided that these primers are designed so that they can amplify any of one or more CpG sequences present in the region between positions -477 and -747 in the TWIST1 promoter region. When, for example, CGCG present at positions 57 to 60 in SEQ ID NO: 1 is amplified, a combination of the primers represented by SEQ ID NOS: 2 and 3 or a combination of the primers represented by SEQ ID NOS: 4 and 5 can be specifically exemplified as described in Examples; a combination of the primers represented by SEQ ID NOS: 4 and 5 can be particularly preferably presented. Moreover, preferred examples of the methylation-specific restriction enzyme in the above step can include a restriction enzyme selected from BstUI, AccII, Bsh1236I, BstFNI, FnuDII, MvnI, and ThaI which can recognize CGCG.

Since the present invention relates to a method used for the detection of colorectal tumor, the cells to be tested according to the present invention are suspected of colorectal tumor cells and not particularly limited in the location and origin thereof. However, for example, the cells are preferably cells derived from tissue suspected of harboring colorectal cancer or a precancerous lesion by endoscopy or the like when emphasis is placed on the sensitivity of detection and are suitably cells contained in the feces, cells contained in a colorectal wash, or cells contained in blood when emphasis is placed on the simplicity of detection. Alternatively, cells contained in serum, urea, sputum, pancreatic juice, bile, seminal fluid, saliva, cerebrospinal fluid, or the like are also suitable.

Examples of the colorectal tumor to be detected according to the present invention can include cancer and adenoma occurring in the colorectum or the digestive tract just proximal thereto; suitable examples thereof are cecal cancer, colon cancer, rectal cancer, cecal adenoma, colon adenoma, and rectal adenoma. Examples thereof are also intended to include anal cancer and anal adenoma.

The colorectal tumor determination kit of the present invention is not particularly limited provided that it is a kit for measuring the degree of methylation of CpG sequence in -477 to -747 of TWIST1 gene in the genome sequence of a cell to be tested and comprises a reagent for extracting genomic DNA, a reagent for converting unmethylated cytosine to uracil, a primer set for amplifying TWIST1 gene promoter region in the genomic DNA or a partial gene fragment thereof containing the CGCG sequence located at positions 57 to 60 in the nucleotide sequence represented by SEQ ID NO: 1, and a reagent for analyzing the methylation pattern. The specific constitution of the kit can comprise, for example, (1) reagents and equipment in the step of extracting DNA, including reagents and equipment for directly extracting DNA from cells; deparaffinization reagents and equipment for extracting DNA from a paraffin specimen and the like; and reagents and equipment for extracting DNA from stool, (2) reagents and equipment in the step of discriminating methylated DNA, such as reagents and equipment for converting unmethylated cytosine to uracil, suitably sodium bisulfite, more suitably sodium hydrogen sulfite and a buffer solution for use in the reaction and the like, (3) reagents and equipment for performing the methylation-specific PCR, i.e., a primer set enabling the amplification of a targeted methylated CGCG region, a heat-resistant DNA polymerase, a buffer solution for the polymerase, and the like, and (4) a methylation-specific restriction enzyme, a buffer solution for the reaction, and the like. As reagents and the like necessary for the above steps, in addition to techniques disclosed as ordinary methods in papers and the like, commercially available kits, for example, All Prep DNA/RNA Mini Kit (Qiagen, Hilden, GERM), QIAamp DNA Micro Kit (from Qiagen) for extracting DNA from a paraffin specimen and the like, QIAamp Stool DNA Isolation Kit (from Qiagen) for extracting DNA from stool, may be used for DNA extraction, and reagents such as, for example, AmpliTaq Gold (from Applied Biosystems) can be used for methylation PCR.
Examples of the present invention will be presented below. However, the present invention is not intended to be limited only to these Examples.

### Example 1

### (Extraction of DNA from Surgically Resected and Frozen Specimen)

The extraction of DNA from surgically resected and frozen specimens was carried out from 252 specimens of normal colorectal mucosa and 320 specimens of colorectal cancer using All Prep DNA/RNA Mini Kit (Qiagen, Hilden, GERM). 600 µl of RLT plus buffer included with the product was added to a 2-ml tube, to which 6 µl of βmercaptoethanol was then added. Each surgically resected and frozen specimen was added thereto, to which one Stainless Steel Bead (5 mm) (from Qiagen) was then added to ultrasonically homogenize the specimen at 30 Hz for 10 minutes using Qiagen Mixer Mill MM300 (from Qiagen). The homogenized lysate was placed in an All Prep DNA spin column (from Qiagen) and centrifuged at 10,000 rpm for 30 seconds. A new All Prep DNA spin column was set in a 2-ml collection tube and incubated at room temperature or 4°C.
500 µl of AW1 buffer (included with the product) was added to the incubated DNA spin column, which was then centrifuged at 10,000 rpm for 30 seconds. After discarding only the waste liquid, the column was set in a 2-ml collection tube. Then, 500 µl of AW2 buffer (included with the product) was added thereto, which was then centrifuged at 15,300 rpm for 2 minutes. The column was transferred to a new 1.5-ml tube. 100 µl of EB buffer (included with the product) was directly added to the column membrane, which was then incubated at room temperature for 1 minute, followed by centrifugation at 10,000 rpm for 1 minute to elute DNA.

### (Preparation of Control Sample)

Placenta-derived DNA (Sigma, St. Louis, MO) treated with SssI methylation transferase (New England Biolabs, Beverly, MA) was used as a positive control for methylated allele. To a solution containing 10 µg of the placenta-derived DNA were added 1 x NES buffer 2 included with the product, 160 µM of S-adenosyl-L-methionine, and 0.2 U (unit) of SssI, which was adjusted to 200 µl with sterilized water. After incubation at 37°C for 2 hours, 3 µl of 32 mM S-adenosyl-L-methionine and 3 µl of SssI were added thereto, which was then incubated at 37°C for 1 hour. Thereafter, an equal amount of phenol/chloroform/isoamyl alcohol (25:24:1, PCI, pH 8.0, from Sigma) was added thereto, which was then suspended for 30 seconds and allowed to stand for 5 minutes. The resultant was centrifuged at room temperature and 14,000 rpm for 5 minutes, and only the supernatant was recovered and transferred to a new tube, to which PCI was then added, followed by performing the above operation once again. To the PCI-treated sample were added 1 µl of 20 mg/ml glycogen (from Roche), 20 µl of 3M sodium acetate, and 500 µl of 100% ethanol, which was then subjected to ethanol precipitation at -80°C for 2 hours. After incubation, the resultant was centrifuged at 4°C and 14,000 rpm for 20 minutes, and the supernatant was removed, followed by rinsing the residue with 70% ethanol. The pellet was dried by vacuum drying, and DNA was dissolved in 100 µl of distilled water to make a positive control for methylated allele.

In contrast, DNA extracted from peripheral blood lymphocytes was used as a positive control for unmethylated allele. Using a blood-collecting vessel containing an anticoagulant EDTA-2Na, 0.2% NaCl was added to peripheral blood collected thereinto, which was then mixed by inversion and centrifuged at 2,500 rpm for 5 minutes. The supernatant was removed with an aspirator in such a manner that white blood cells precipitated on the vessel bottom are not aspirated, followed by repeating the operation until the pellet (white blood cells) becomes white. The whitened pellet was transferred to a 1.5-ml tube and subjected to nucleic acid extraction using a nucleic acid-extracting agent, SepaGene (Sanko Junyaku Co., Ltd.). The extraction method was according to the direction for use. The pellet of nucleic acid obtained by the extraction was air-dried, to which 100 µl of distilled water was then added for dissolution. The solution was used as a positive control for unmethylated allele.

### (Treatment of DNA with Sodium Hydrogen Sulfite)

To modify methylated DNA to subject to analysis using a sodium bisulfite PCR method, it was treated with sodium hydrogen sulfite. To 2 µg of the DNA purified above was added distilled water to a total amount of 50 µl, to which 5.5 µl of 2 M NaOH was then added, followed by incubation at 37°C for 10 minutes. 30 µl of 10 mM hydroquinone (Sigma) and 520 µl of 3 M sodium hydrogen sulfite (pH 5.0, Fisher Scientific) were added thereto, which was then incubated at 50°C for 16 hours under shielding the light. The DNA sample treated with sodium hydrogen sulfite was purified using DNA Cleanup Kit (Promega). The purification method was according to the direction for use. 5.5 µl of 3 M NaOH was added to the purified DNA, which was then incubated for 5 minutes. 1 µl of 20 mg/ml glycogen, 33 µl of 10 M ammonium acetate, and 260 µl of 100% ethanol were added thereto, which was then subjected to ethanol precipitation. After ethanol precipitation, DNA was pelletized under vacuum drying. 100 µl of distilled water was added to the pellet, which was then vortexed for 30 minutes for dissolution. The solution was allowed to stand at 4°C overnight and dissolved to make a sodium bisulfite-treated DNA sample. Treatment with sodium hydrogen sulfite converts cytosine in unmethylated CpG to uracil, while this conversion does not occurs for methylated cytosine. This enables the detection of the difference between the fact that a PCR product obtained using methylated DNA as a template is cleaved by restriction enzyme treatment to be described later and the fact that a PCR product obtained using unmethylated DNA as a template is not cleaved by the treatment.

### (Quantitative Analysis of Methylation)

For the quantitative analysis of methylation, COBRA (Combined Bisulfite Restriction Analysis) assay was carried out. Using AmpliTaq Gold DNA Polymerase (Applied Biosystems) as polymerase, 20 µl of a reaction solution was prepared which comprises 2 µl of GenAmp 10 x Buffer II (included with the product), 2 mM magnesium chloride, 800 nM dNTPs, 500 nM primers (described in Table 1, SEQ ID NOS: 2 and 3), 2 µl of sodium bisulfite-treated DNA, and 0.5 U of the polymerase. The details of the primers are shown in Table 1 below and SEQ ID NOS: 2 and 3. The temperature conditions of PCR are also shown in Table 2 below. The PCT product was identified by 3% agarose gel electrophoresis.

**[Table 1]**

| Primers used for quantitative determination of methylation of TWIST1 gene | | |
|---|---|---|
| Primer | Sequence | Size (bp) of Product |
| TWIST1 Forward (SEQ ID NO: 2) | 5'-TGTGTAGAAGTTGTTGTTATTG-3' | 79 |
| TWIST1 Reverse (SEQ ID NO: 3) | 5'-CRAAAAAAACTATCCTAACC-3' | |

**[Table 2]**

| PCR for quantitative analysis of methylation |
|---|
| 95°C, 10 min |
| 40 cycles |
| \| 95°C, 30 sec |
| \| 55°C, 30 sec |
| \| 72°C, 30 sec |
| 72°C, 4 min |
| |

8 µl of a sample in which a desired PCR product was identified was dispensed in a new tube, to which 10 U of the restriction enzyme BstUI (New England Biolabs) and 1 µl of 10 x NEB Buffer 4 included with the product were then added to prepare a total 10 µl of a reaction solution, followed by incubation at 60°C for 2 hours. BstUI is a restriction enzyme recognizing and cleaving the base sequence 5'-nnnCG↓CGnnn-3'. In the sodium bisulfite-treated DNA, CGCG remains unchanged even after being sodium bisulfite-treated if methylation is present in all cytosine of CGCG; a PCR product having this sequence is cleaved by the restriction enzyme BstUI. In contrast, CGCG is converted to any of TGTG, CGTG and TGCG after being sodium bisulfite-treated if methylation is absent in any or all cytosine thereof; PCR products having each of the three sequences are not cleaved by BstUI. The use of such properties enables the presence or absence of methylated DNA and its proportion to be visualized by the presence or absence of a band and its density. The restriction enzyme-treated DNA samples were subjected to electrophoresis using a 4% agarose gel containing ethidium bromide, and the photograph of the gel was analyzed using Fujifilm Multi Gauge V3.0 software (from Fujifilm Corporation) to quantitatively determine the methylated DNA and the unmethylated DNA.

### (Results)

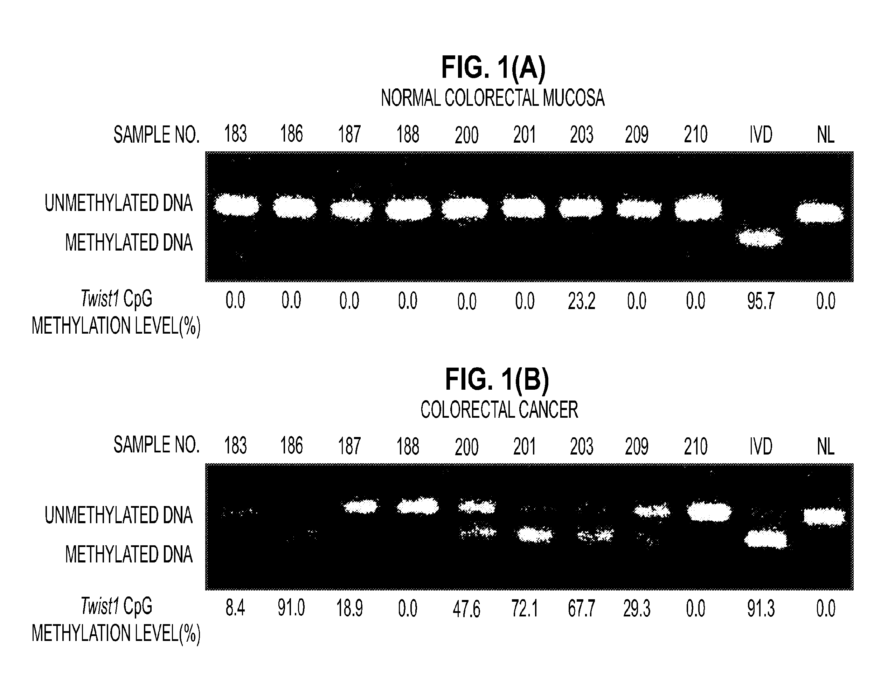

Figure 1 shows the results of the COBRA assay. In the figure, the upper stage shows the electrophoresis of BstUI-degraded fragments of PCR products obtained using sodium bisulfite-treated genomic DNAs derived from normal colorectal mucosa cells as templates and the lower stage shows the electrophoresis of BstUI-degraded fragments of PCR products obtained using sodium bisulfite-treated genomic DNAs derived from colorectal cancer cells as templates. The primers used in this Example targets the methylation of CGCG at positions -688 and -691 present in the sequence containing the regulatory region of TWIST1 gene and the vicinity thereof at positions -477 and -747 for detection. Each unmethylated DNA was not cleaved by BstUI and showed a band on the higher molecular weight side (indicated by "unmethylated DNA" in the figure), and each methylated DNA showed a band on the lower molecular weight side (indicated by "methylated DNA" in the figure); the densities (fluorescence intensities) of the bands reflected the methylation/non-methylation ratio of the target in the genome. The numbers below each lane each represent a number expressing the methylation level of TWIST1 CpG as % based on the whole CpG. Theoretically, the number has a value of 100 if all CpG are methylated and 0 if all CpG are unmethylated. The numbers above each lane represent the numbers of the samples. IVD located at the right of each sample lane represents a positive control for methylation and NL represents a positive control for non-methylation.

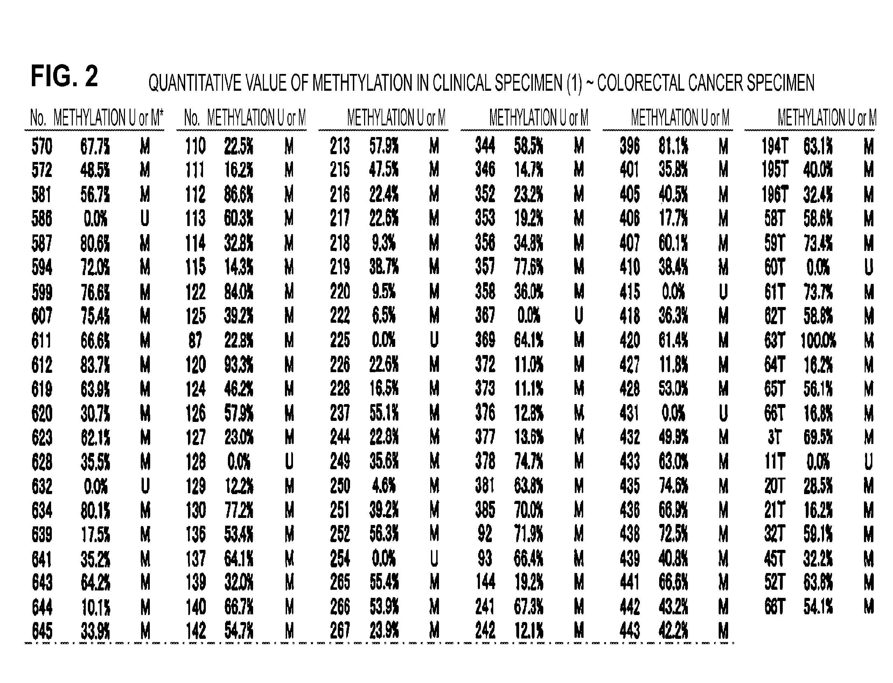

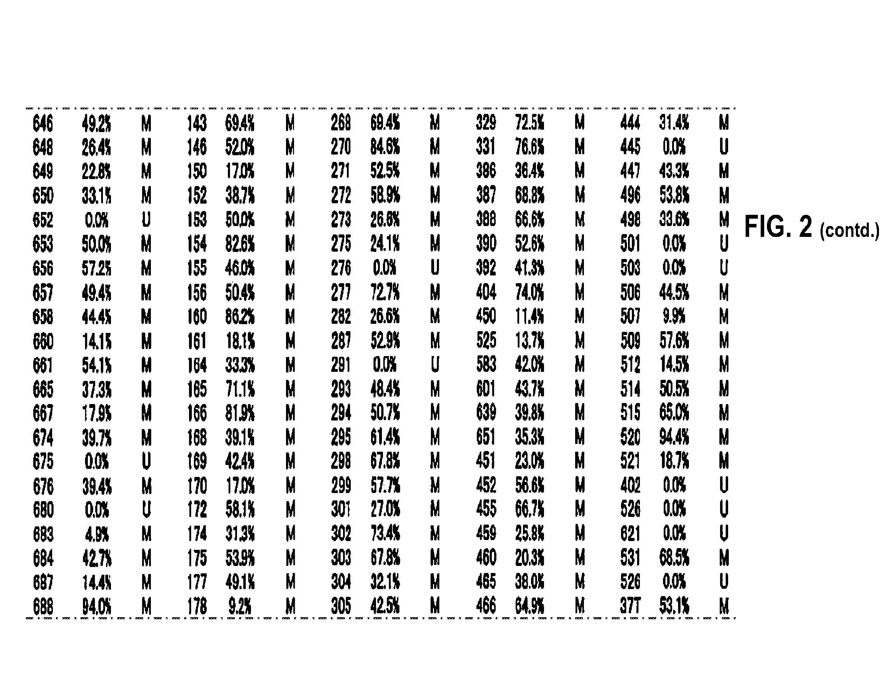

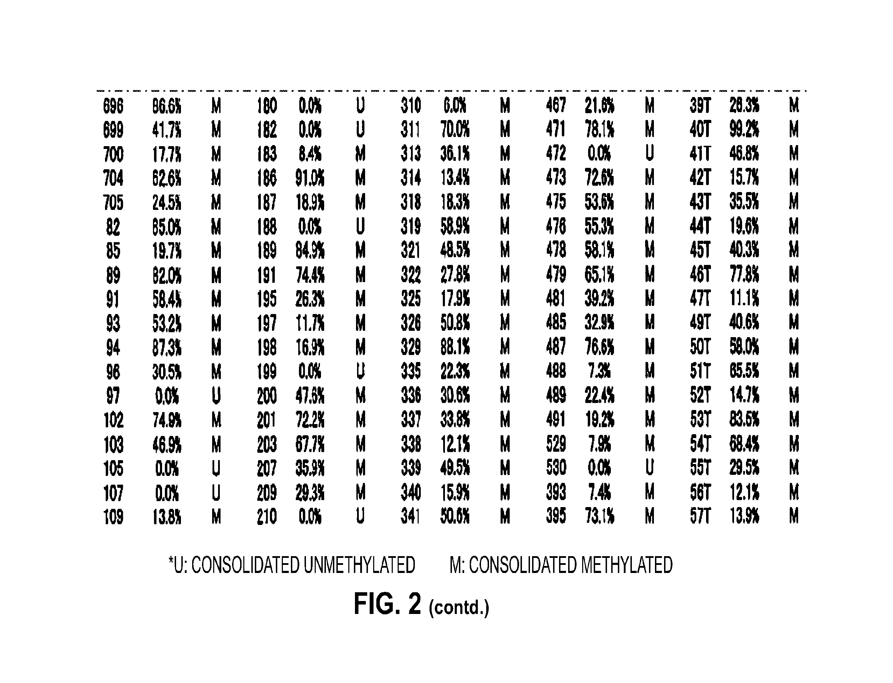

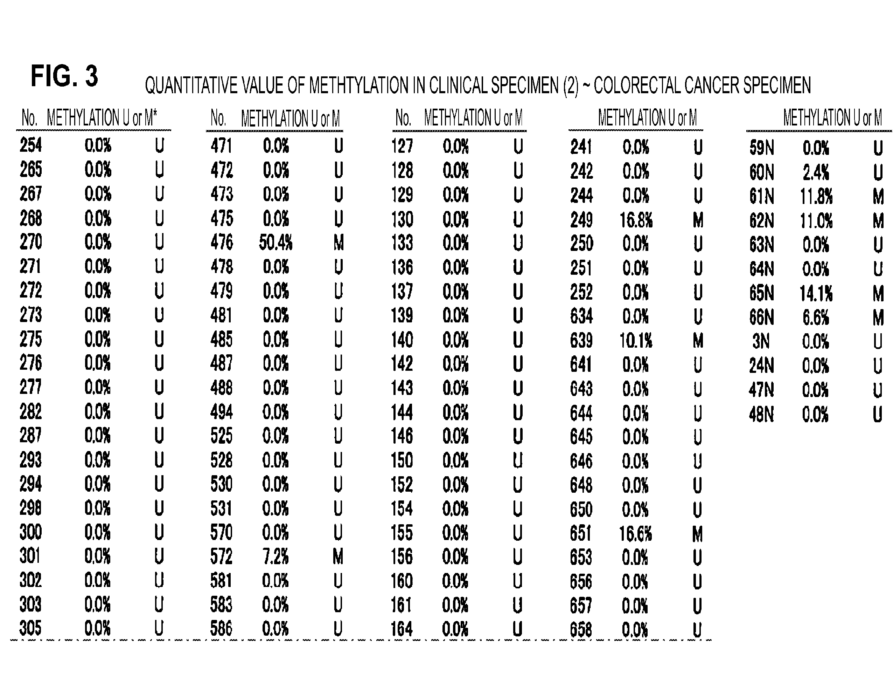

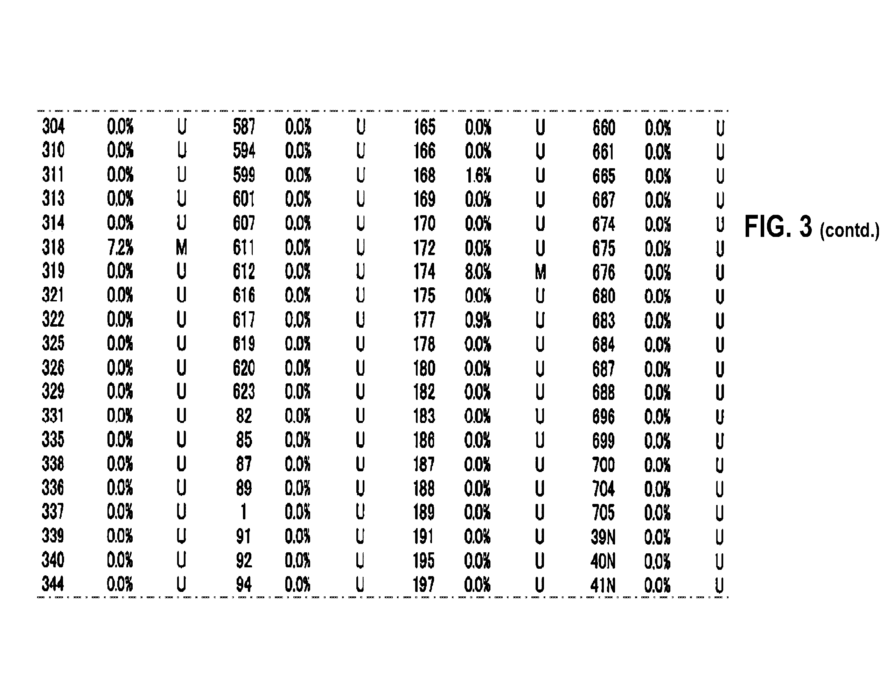

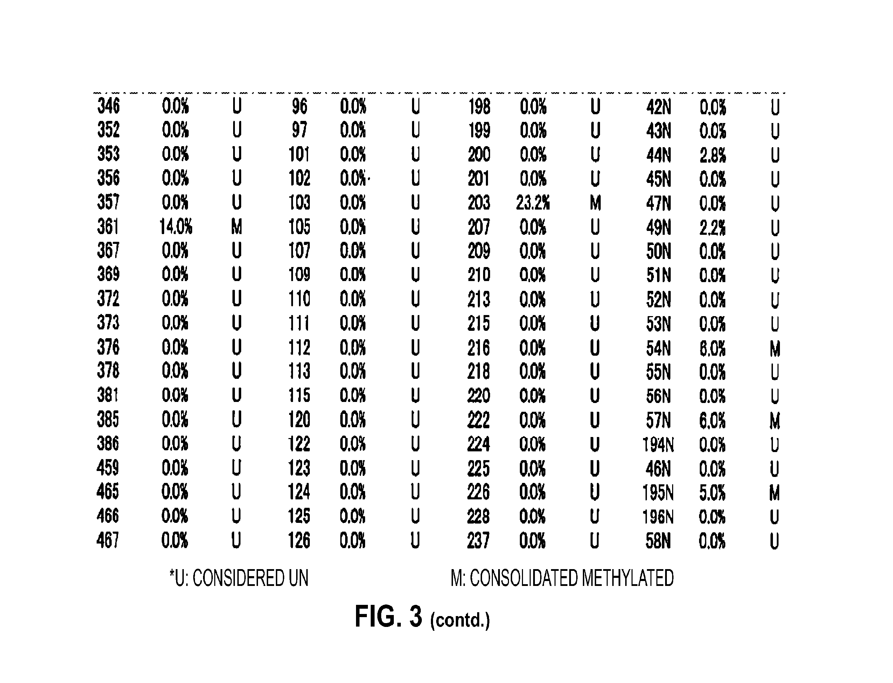

For the normal colorectal mucosa in Figure 1A, some of representative photographs are shown; unmethylated DNA was predominant and the median of the levels of methylation was 0.0%. In contrast, for the colorectal cancer in Figure 1B, the ratio of methylated DNA increased and the median of the methylation levels had a high value of 40.1%. IVD located at the right of the sample lane of each of Figures 1 A and B represents a positive control for methylation and NL represents a positive control for non-methylation.
Methylation data from all specimens of normal colorectal mucosa and colorectal cancer are shown in Figure 2 (colorectal cancer) and Figure 3 (normal colorectal mucosa). In the figures, "No." represents the numbers of the specimens; "methylation" (expressed as %) represents the percentage of methylation calculated from the fluorescence intensity of bands; and "U or M" represents the determination of methylation (U = not methylated, M = methylated) when a cutoff value to be described later is applied.

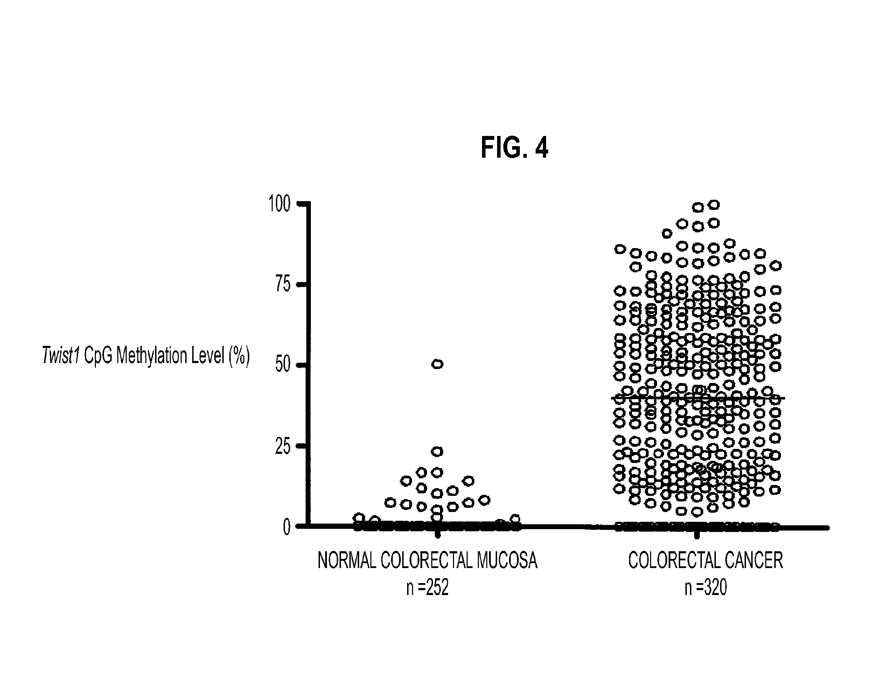

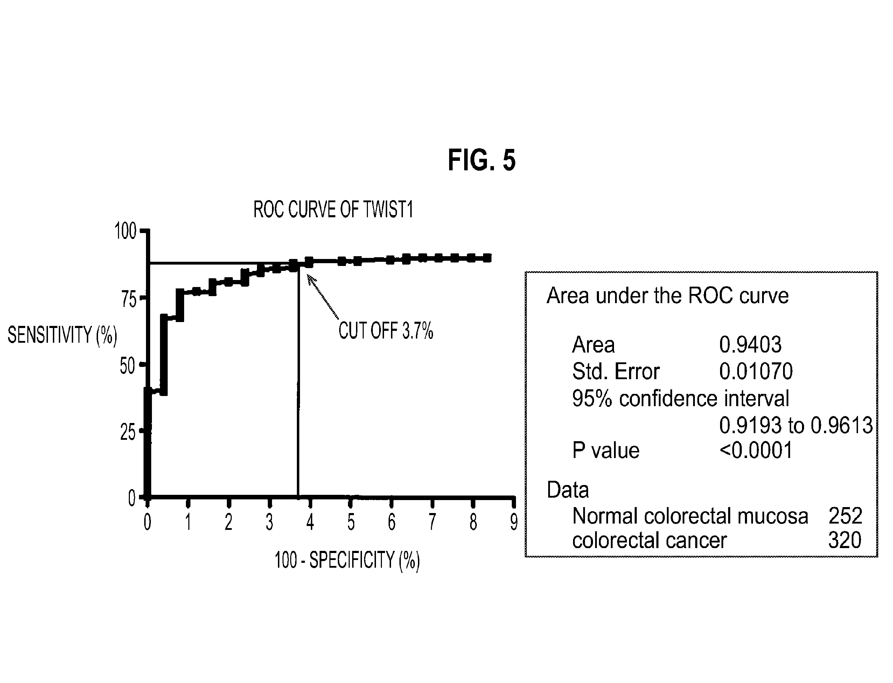

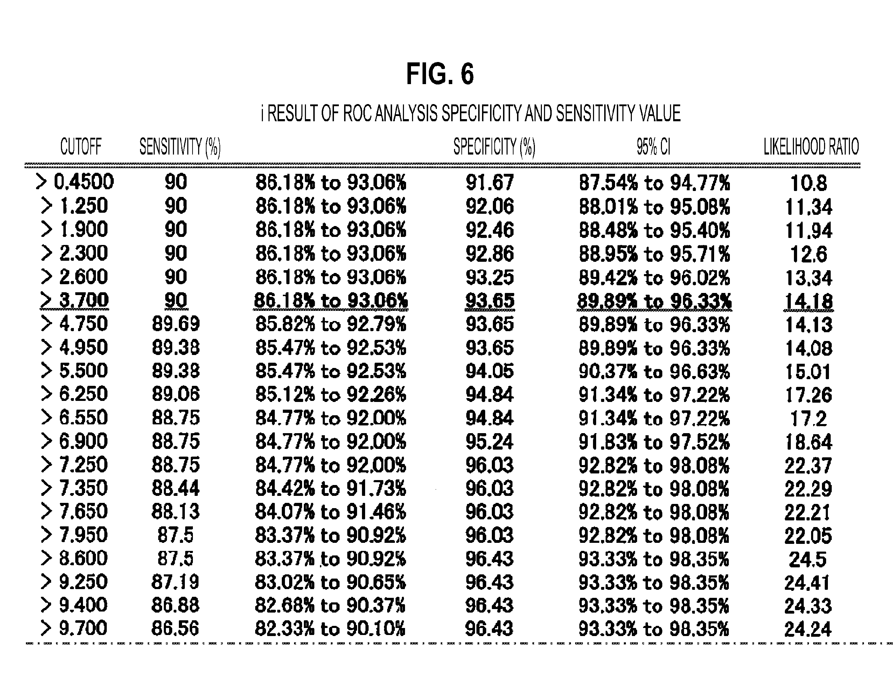

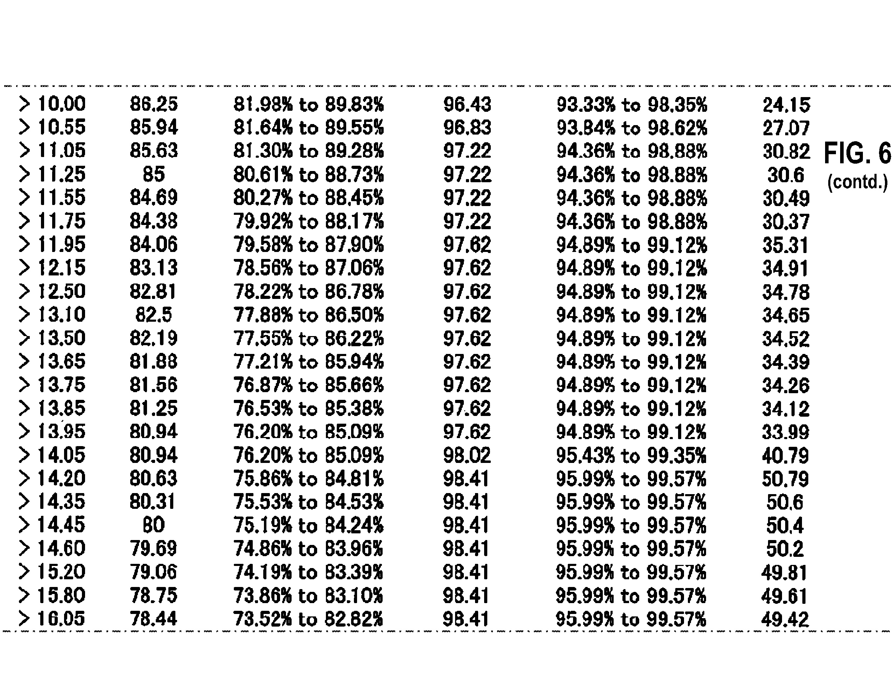

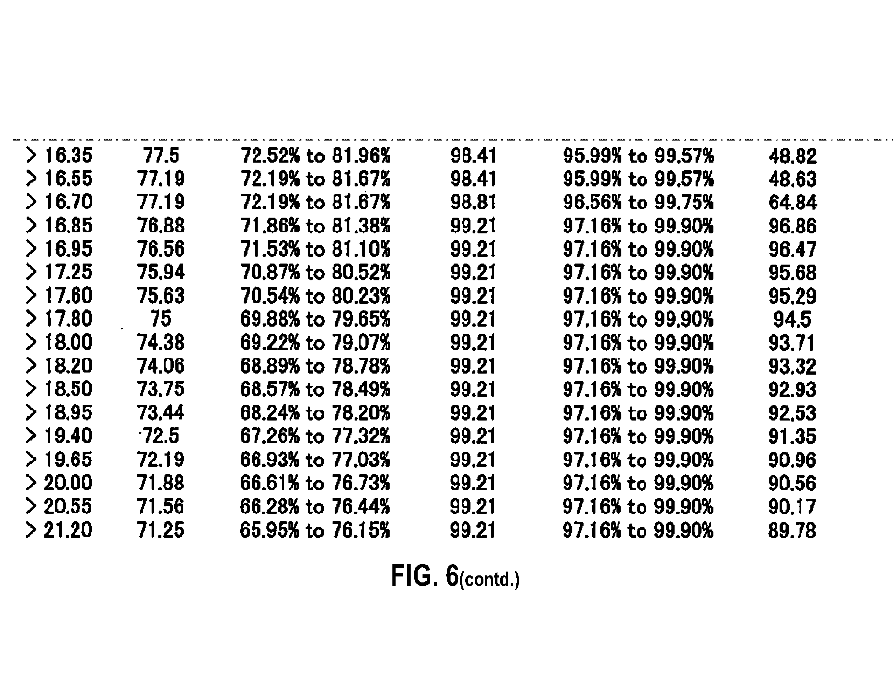

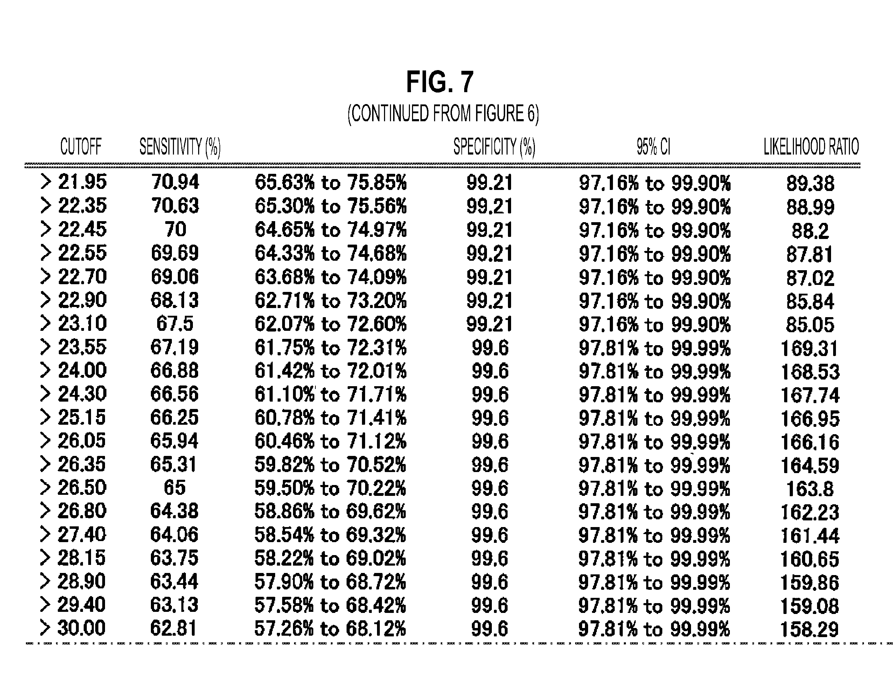

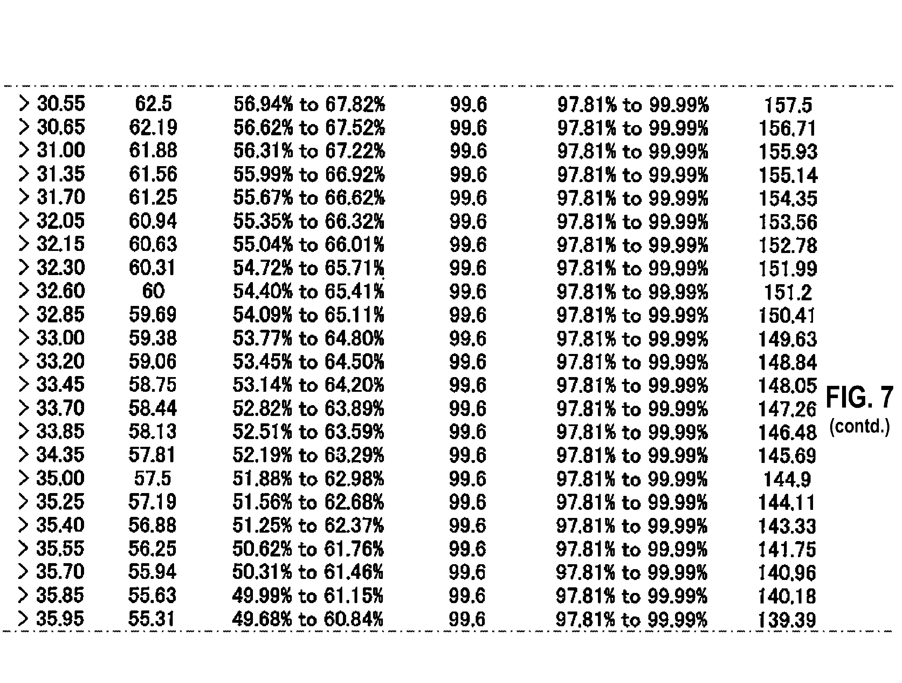

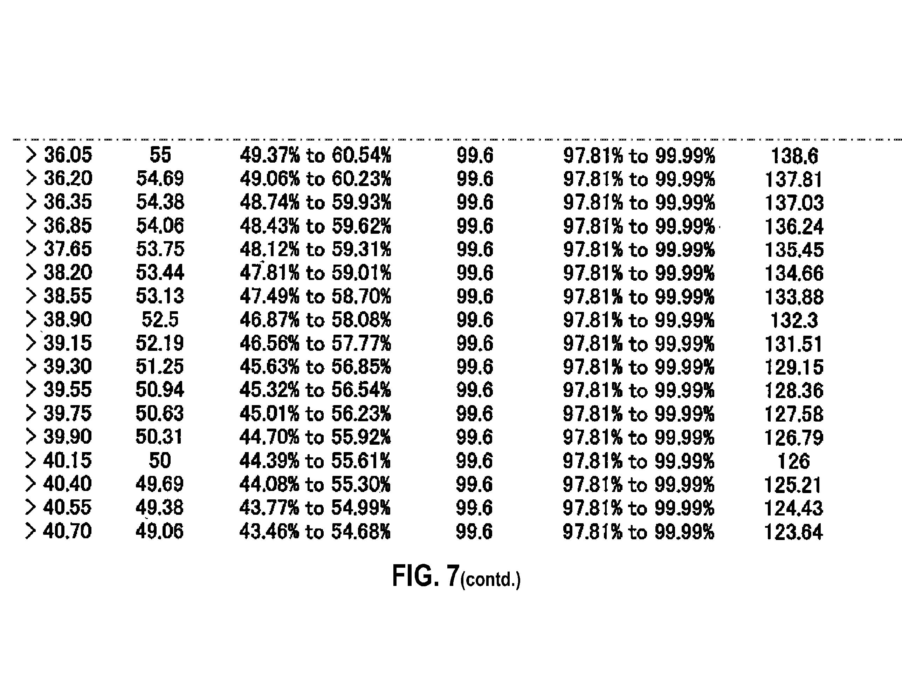

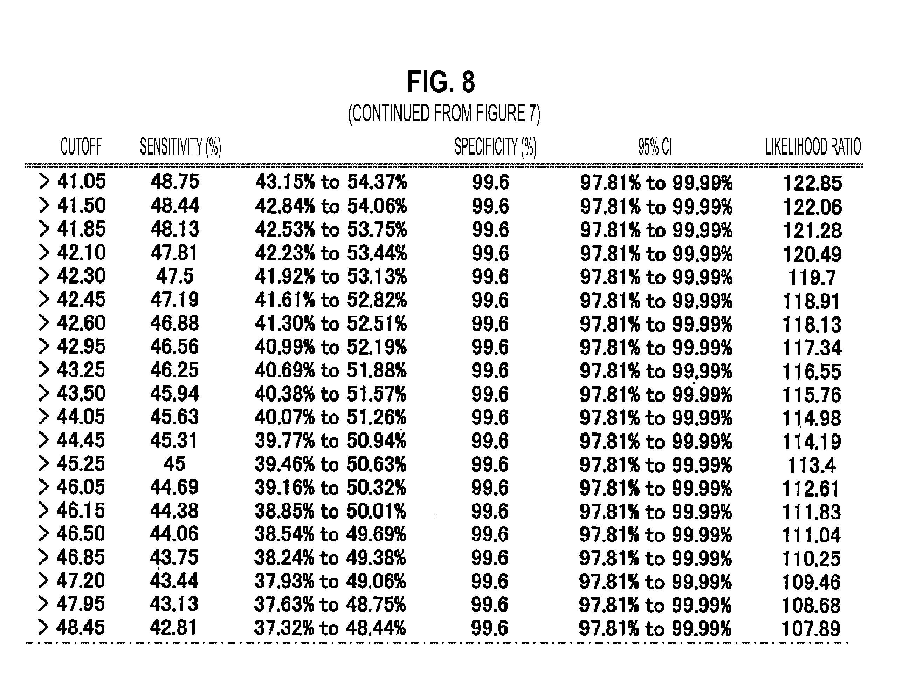

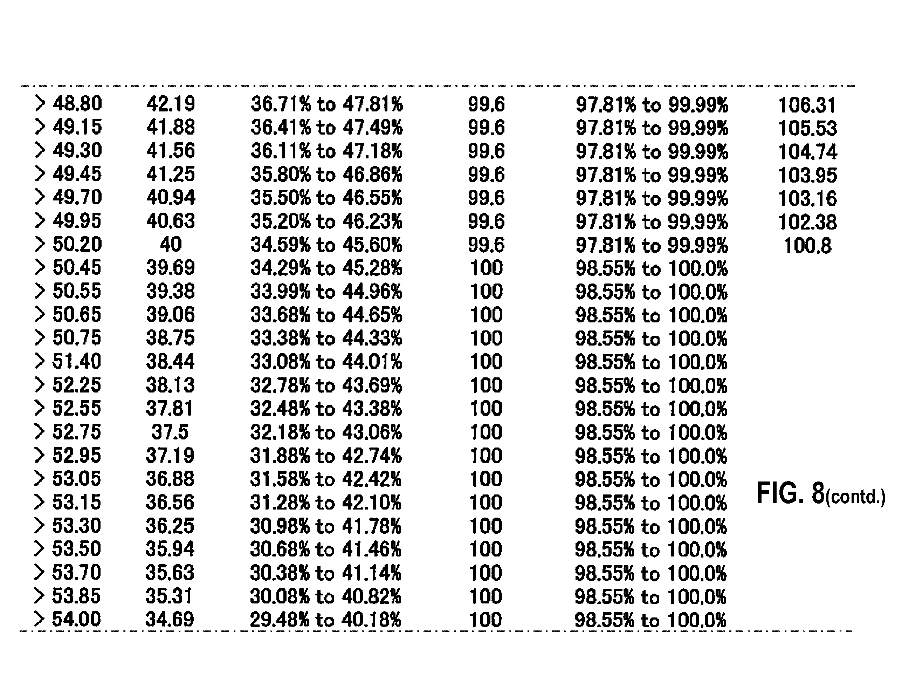

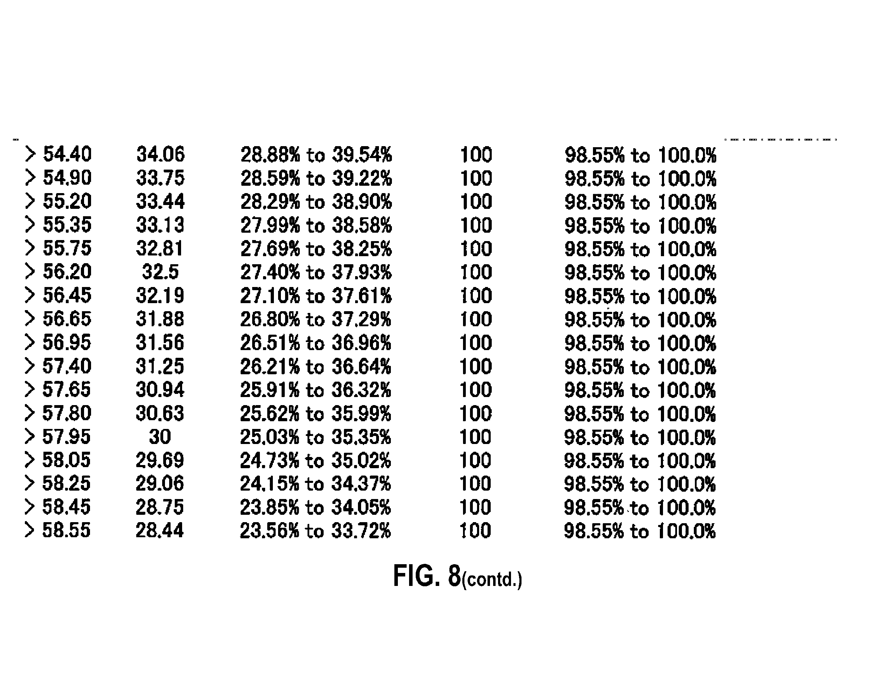

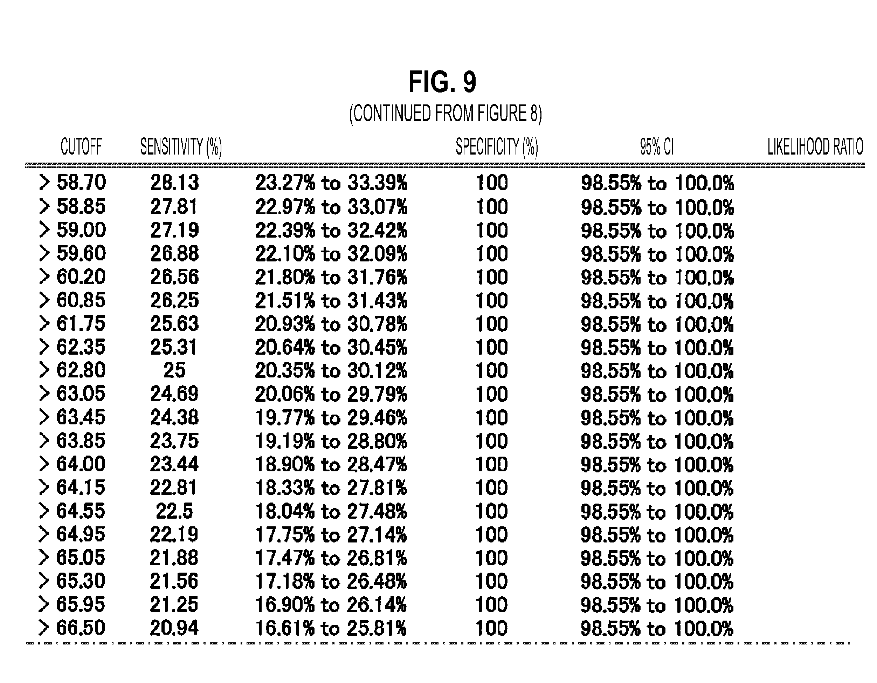

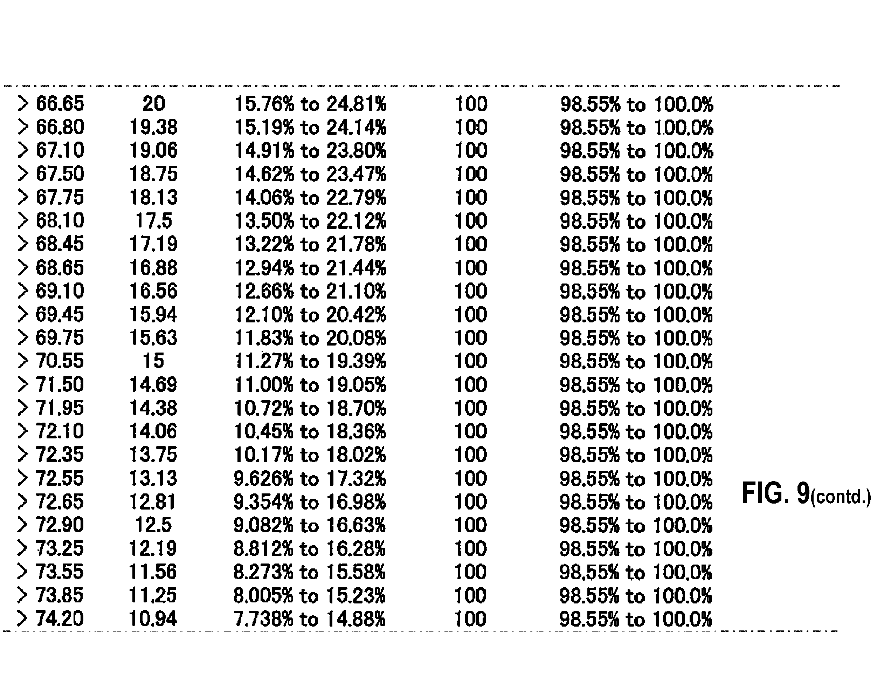

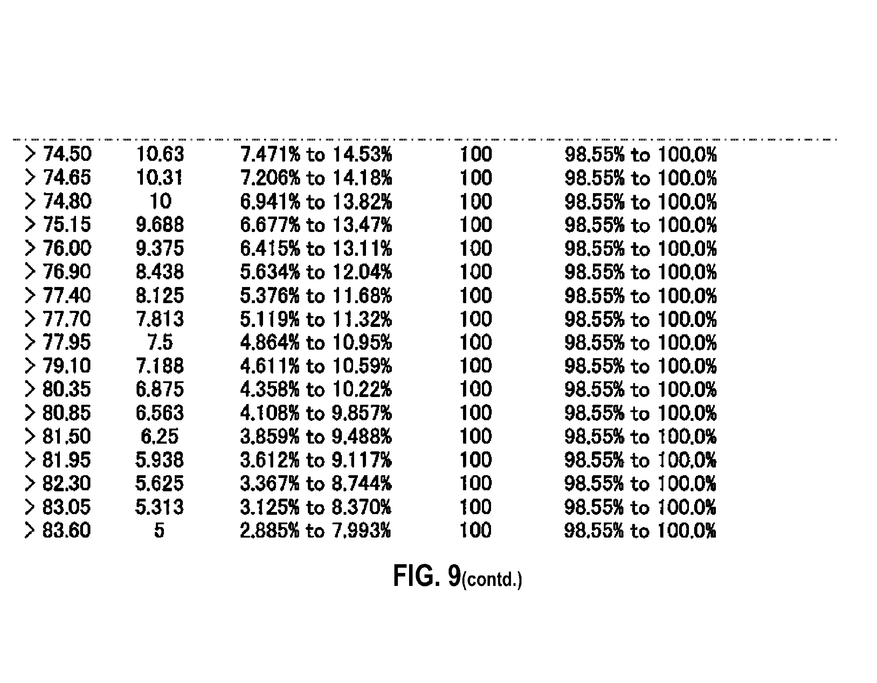

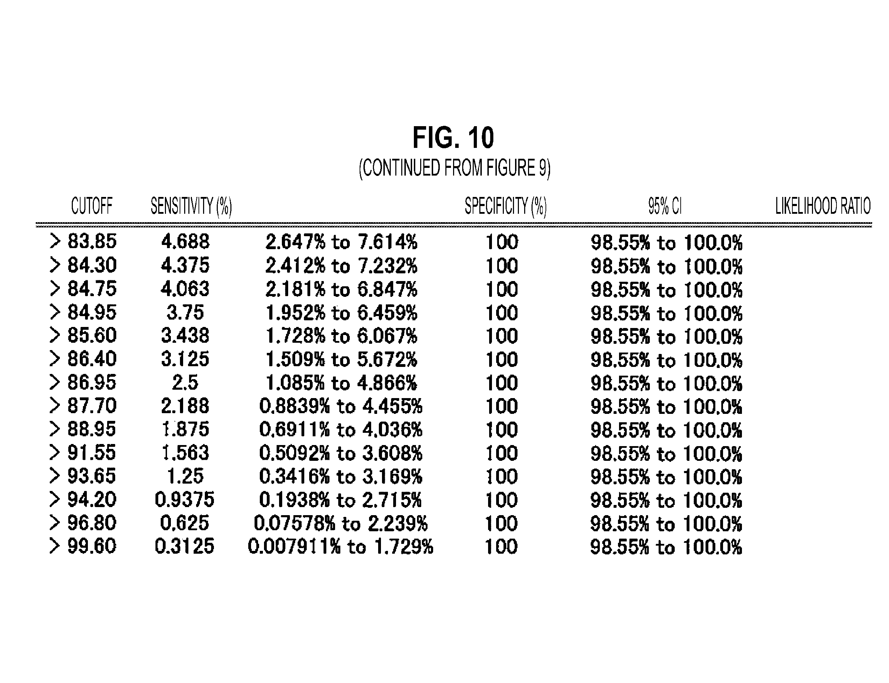

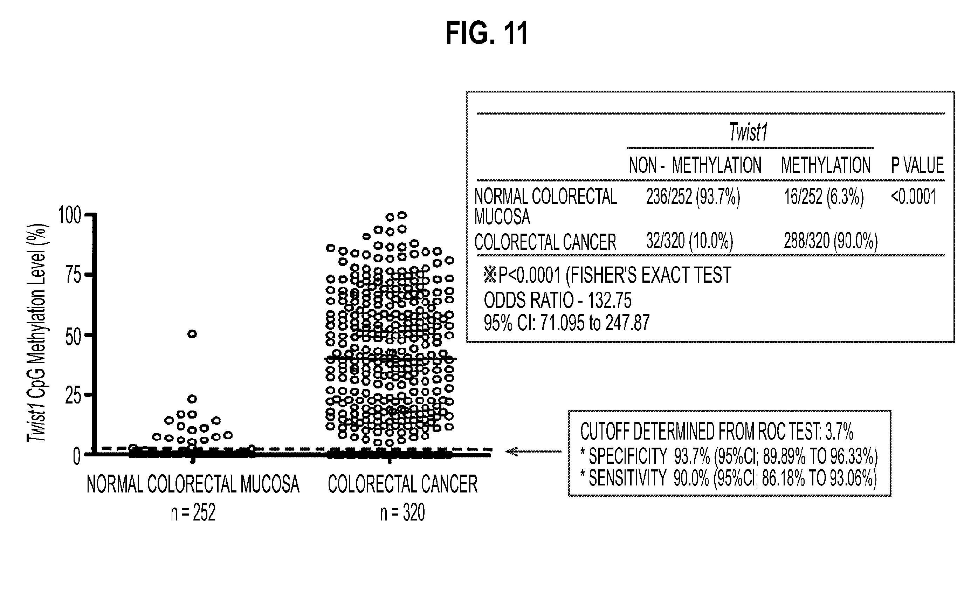

Using genomic DNA extracted from 252 specimens of normal colorectal mucosal tissue and 320 specimens of colorectal cancer tissue, the same methylation level analysis as that described above was carried out. Figure 4 shows the methylation levels in normal colorectal mucosa and colorectal cancer. The ordinate axis in the graph represents the methylation level of TWIST1 CpG in %; each point (o) represents one specimen. As shown in the graph, the genomic DNA derived from normal colorectal mucosa had a methylation level of 5.0% or less in almost all of the specimens, whereas the genomic DNA derived from colorectal cancer had a remarkably increased methylation level. The median for the normal colorectal mucosa was 0.0%, whereas the median for colorectal cancer was 40.1% (P < 0.0001). These results strongly suggested the possibility of distinguishing colorectal cancer based on the methylation level of TWIST1 gene; thus, an ROC curve (receiver operating character curve) was prepared using the above-described 252 specimens of normal colorectal mucosa and 320 specimens of colorectal cancer. Figure 5 shows the ROC curve prepared. In the graph, the ordinate axis represents the detection sensitivity and the horizontal axis represents 100.0% specificity. The data used for the ROC analysis were also shown in Figures 6 to 10. In the figures, "Cutoff" represents a cutoff value; "Sensitivity", the detection sensitivity (%); "95% CI", 95% confidence interval; and "Specificity", specificity (%), "Likelihood ratio" shows the ratio of likelihood. Figures 6 to 10 are divisions of data whose cutoff values are from 0.45 to 99.80; the likelihood ratios after Cutoff = 50.45 are not determined. From the graph and Figures 6 to 10, the methylation level = 3.7% at which the sensitivity and specificity had optimal values (shown in boldface numbers and underlines in Figure 6) was considered to be a cutoff value. Figure 11 is a graph in which the cutoff value was applied to the graph in Figure 4. The cutoff value (3.7%) is shown in dotted lines on the graph, and the ratios of methylated/unmethylated DNA when the cutoff value is applied are shown in a box at the right top of the graph. When the cutoff value of 3.7% was applied, the percentage of methylated DNA in colorectal cancer was 90.0% (288/320), while that in normal colorectal mucosa had an extremely low value of 6.3% (16/252). The sensitivity and specificity derived from the value were 90.0% and 93.7%. The positive hitting ratio was 94.7% (288/304) and the negative hitting ratio was 88.1% (236/268).

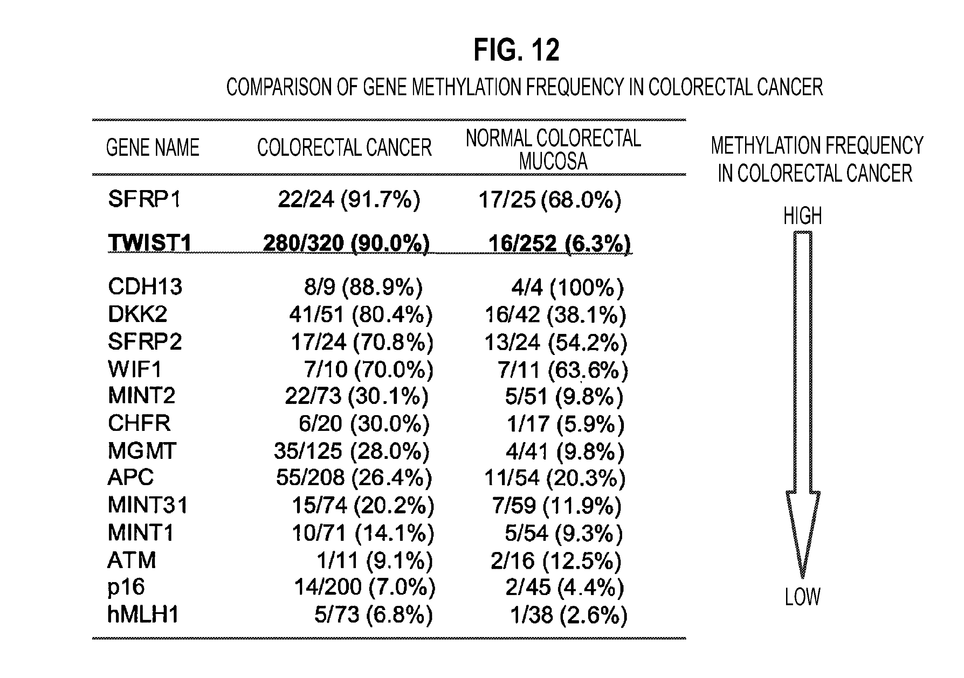

The methylation frequency of TWIST1 gene in colorectal cancer and normal colorectal mucosa according to Examples of the present invention is shown in Figure 12 by comparison with the results obtained using other genes previously reported for colorectal cancer. In the figure, "Gene Name" represents the abbreviated name of the gene reported; "Colorectal Cancer", the methylation frequency (the number and percentage of methylated genes) of each gene in colorectal cancer cells; and "Normal Colorectal Mucosa", the methylation frequency (the number and percentage of methylated genes) of each gene in normal colorectal mucosa. The genes are arranged in order of decreasing methylation frequency (%) in colorectal cancer, which is indicated on the right side of the table. Characters in the row of "TWIST1" are shown in bold type and underlined.
As shown in Figure 12, SFRP gene had a higher methylation frequency (91.7%) than that (90.0%) of TWIST1 gene in colorectal cancer. However, SFRP gene also had a high methylation frequency of 68.0% in normal colorectal mucosa, which is in contrast with the fact that TWIST1 gene had an extremely low methylation frequency of 6.3% in normal colorectal mucosa. Other genes previously reported also show that genes having a high methylation frequency in colorectal cancer also each tend to have a high methylation frequency in normal colorectal mucosa, reflecting a detection specificity problem. The TWIST1 gene provided by the present invention has the property required of a marker that it is highly specifically methylated in colorectal cancer and is evidently superior to conventional markers.

### Example 2

### (Detection of Colorectal Adenoma Using Methylation of TWIST1 Gene as Measure)

Using the same protocol as that in Example 1, it was verified whether the methylation of TWIST1 gene could be a marker for colorectal adenoma as a benign tumor occurring in the colorectum.
189 paraffin-embedded specimens of formalin-fixed colorectal adenoma were subjected to the recovery of only a tumor site by microdissection, followed by DNA extraction using Qiagen DNA FFPE Tissue kit (from Qiagen). The extraction method was according to the protocol included with the product.

### (Preparation of Sample)

Control samples were prepared according to Example 1: a positive control for methylated allele was prepared from placenta-derived DNA (Sigma) treated with SssI methylation transferase (New England Biolabs) and a positive control for unmethylated allele, from DNA extracted from peripheral blood lymphocytes.

### (Treatment of DNA with Sodium Hydrogen Sulfite and Quantitative Analysis of Methylation)

According to Example 1, to modify methylated DNA to subject to analysis using a sodium bisulfite PCR method, it was treated with sodium sulfite. COBRA assay for quantitative analysis of methylation was also performed according to the method of Example 1. However, the primers used newly designed primers as shown in Table 3 below (see SEQ ID NOS: 4 and 5), and the PCR for quantitative analysis of methylation was performed using a program as shown in Table 4 below. It is the purpose of the improvement to also enable the detection of methylation by quantitative PCR assay, and the reason of the improvement is that the evaluation of methylation using the quantitative PCR, if becomes feasible, enables high-throughput analysis.

**[Table 3]**

| Primers used for quantitative determination of methylation of TWIST1 gene | | |
|---|---|---|
| Primer | Sequence | Size (bp) of Product |
| TWIST1 Forward (SEQ ID NO: 4) | 5'-TGTGTAGAAGTTGTTGTTATT-3' | 79 |
| TWIST1 Reverse (SEQ ID NO: 5) | 5'-CRAAAAAAACTATCCTAAC-3' | |

**[Table 4]**

| PCR for quantitative analysis of methylation |
|---|
| 95°C, 10 min |
| 43 cycles |
| \| 95°C, 30 sec |
| \| 55°C, 30 sec |
| \| 72°C, 30 sec |
| 72°C, 4 min |

### (Results)

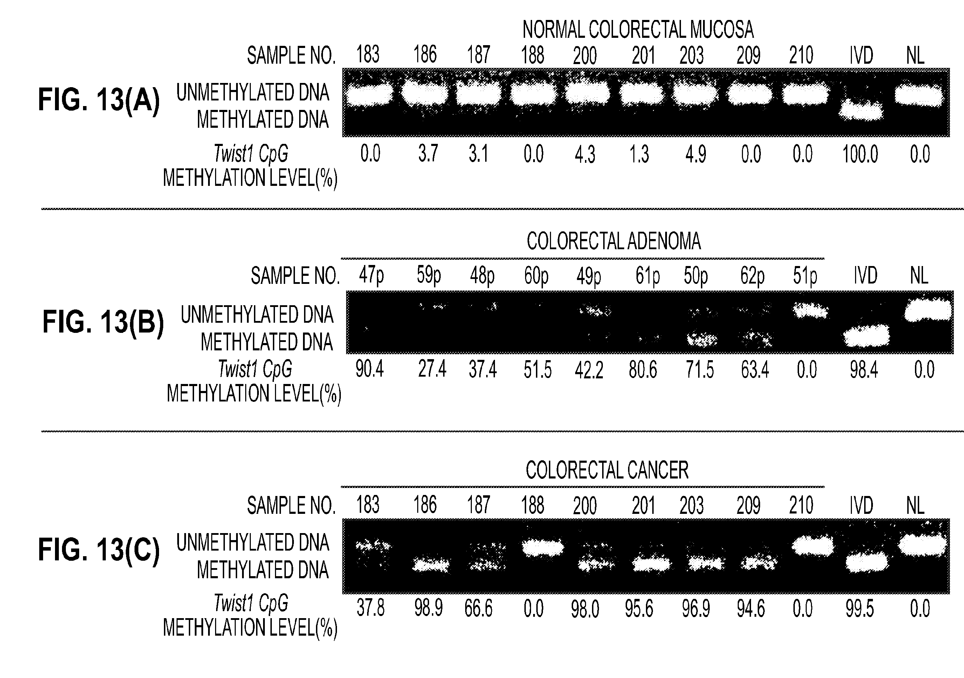

Figure 13 shows the results of the COBRA assay. In the figure, the upper stage (A) shows the electrophoresis of BstUI-degraded fragments of PCR products obtained using sodium bisulfite-treated genomic DNAs derived from normal colorectal mucosa as templates; the middle stage (B) shows the electrophoresis of BstUI-degraded fragments of PCR products obtained using sodium bisulfite-treated genomic DNAs derived from colorectal adenoma as templates; and the lower stage (C) shows the electrophoresis of BstUI-degraded fragments of PCR products obtained using sodium bisulfite-treated genomic DNAs derived from colorectal cancer cells as templates. The primers used in this Example targets the methylation of CGCG at positions -688 and -691 present in the sequence containing the regulatory region of TWIST1 gene and the vicinity thereof at positions -477 and -747 for detection. IVD located at the right lane in the figure represents positive control for methylation and NL represents positive control for non-methylation. Nine representative samples are presented in each of other regions of the lanes.
For the normal colorectal mucosa in Figure 13A, unmethylated DNA was predominant and the median of the levels of methylation was 0.0%. In contrast, for the colorectal adenoma in Figure 13B, the median of the methylation levels had a high value of 25.6% and was approximately half the median of the levels of methylation of 55.7% in colorectal cancer. These results show that the detection method for colorectal tumor provided by the present invention enables the detection of colorectal adenoma as a benign tumor as well as colorectal cancer and that the method enables the degree of progression of the tumor to be detected and estimated by the degree of methylation.

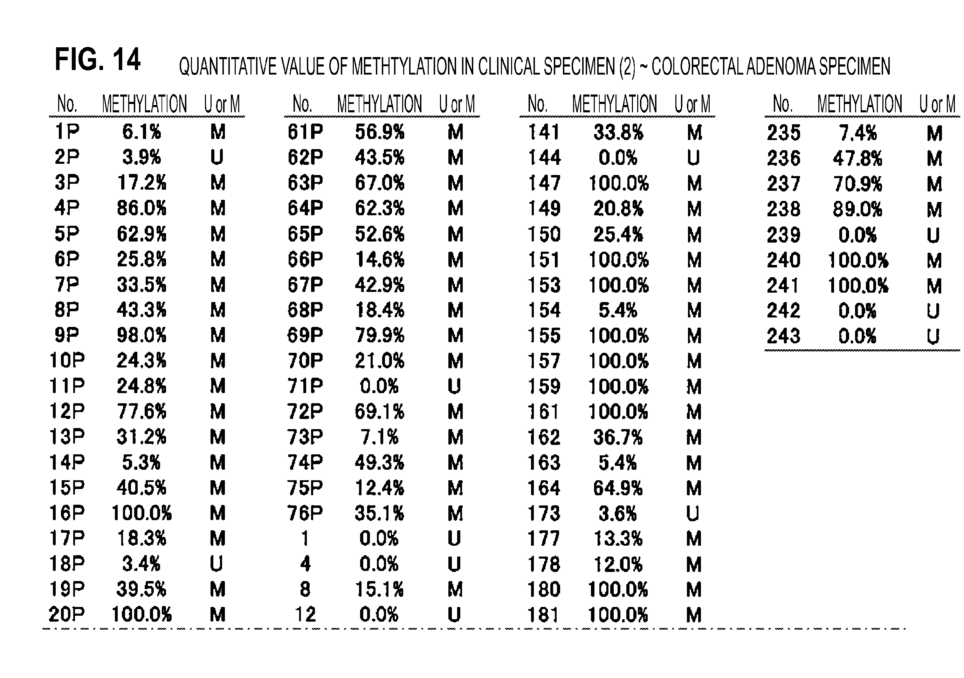

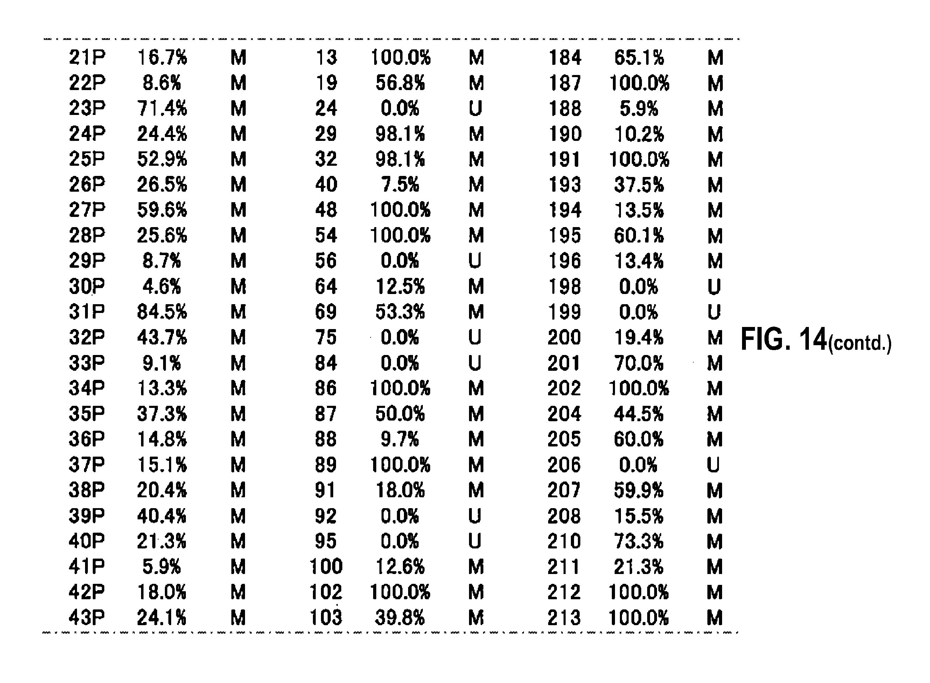

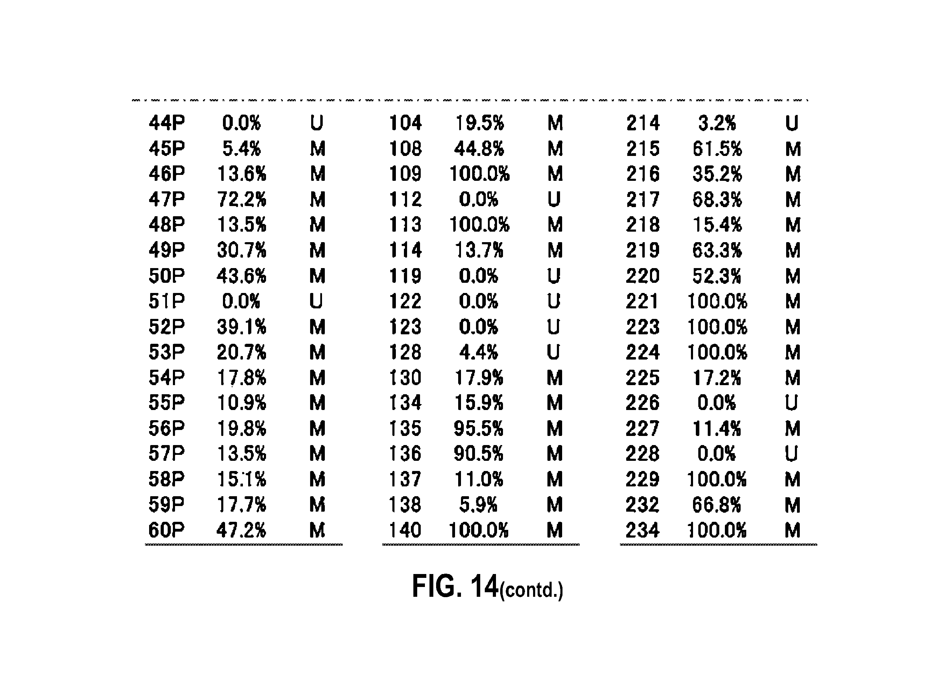

Figure 14 shows methylation data of all 189 specimens of colorectal adenoma in this Example. In the figures, "No." represents the numbers of the specimens; "methylation" (expressed as %) represents the percentage of methylation calculated from the fluorescence intensity ratio between bands; and "U or M" represents the determination of methylation (U = not methylated, M = methylated) when a cutoff value to be described later is applied.

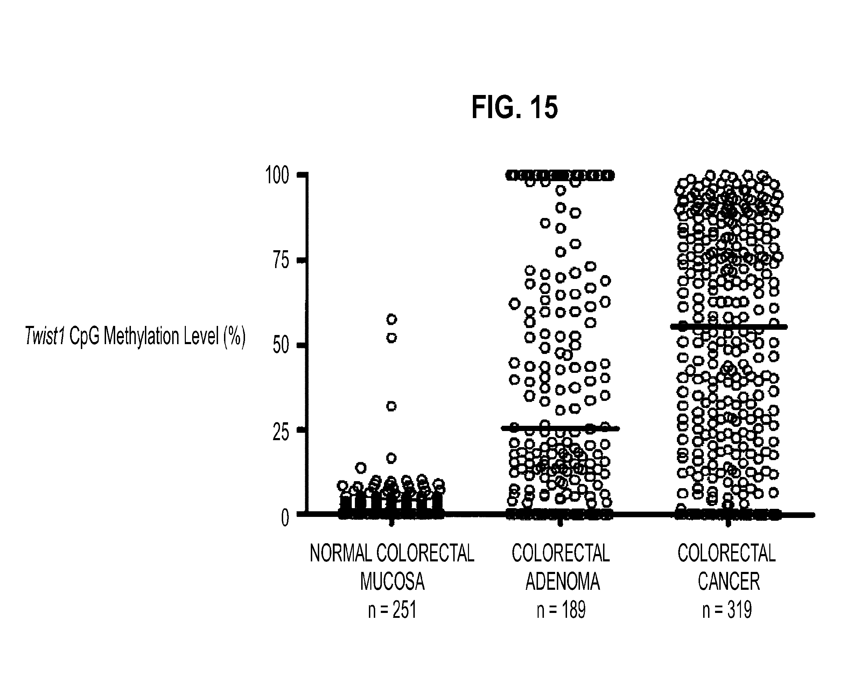

Figure 15 shows the methylation level of colorectal adenoma specimens in comparison with normal colorectal mucosal tissue (251 specimens; one specimen was deleted for experimental reasons) and colorectal cancer tissue (189 specimens). The graph was prepared according to Figure 4 in Example 1, and the data of normal colorectal mucosa and colorectal cancer are common to those in Figure 4. The ordinate axis represents the methylation level (%) of TWIST1 CpG and each point (○) represents one specimen. As shown in the graph, the genomic DNA derived from the normal colorectal mucosa had a methylation level of 5.8% or less in almost all of the specimens, whereas the genomic DNA derived from the colorectal adenoma and colorectal cancer had a high methylation level. The median of methylation levels was 0.0% for normal colorectal mucosa, 25.6% for colorectal adenoma, and 55.7% for colorectal cancer (P < 0.0001).

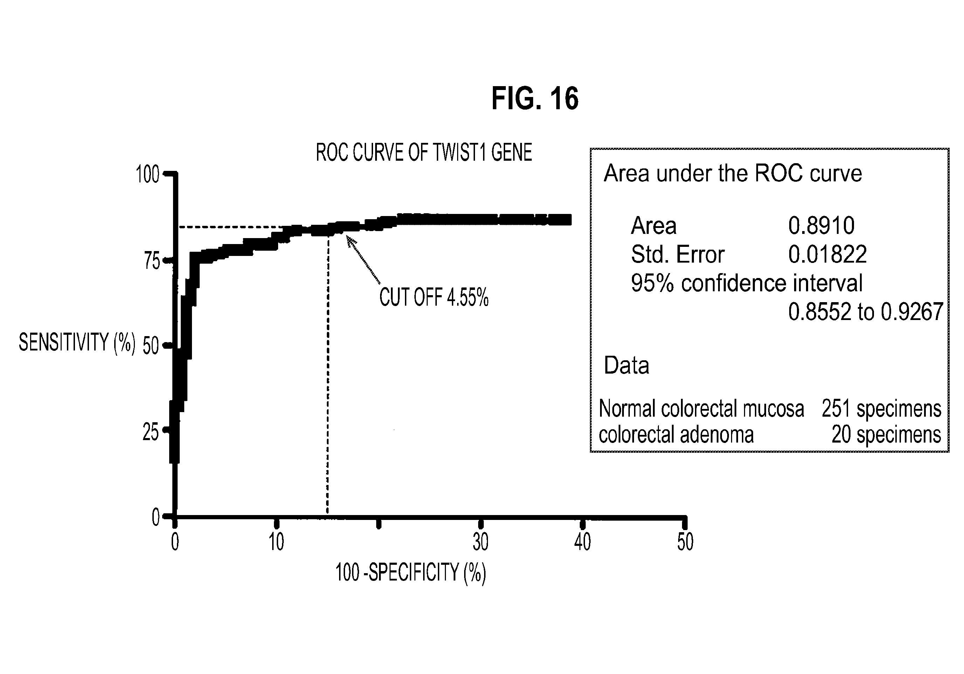

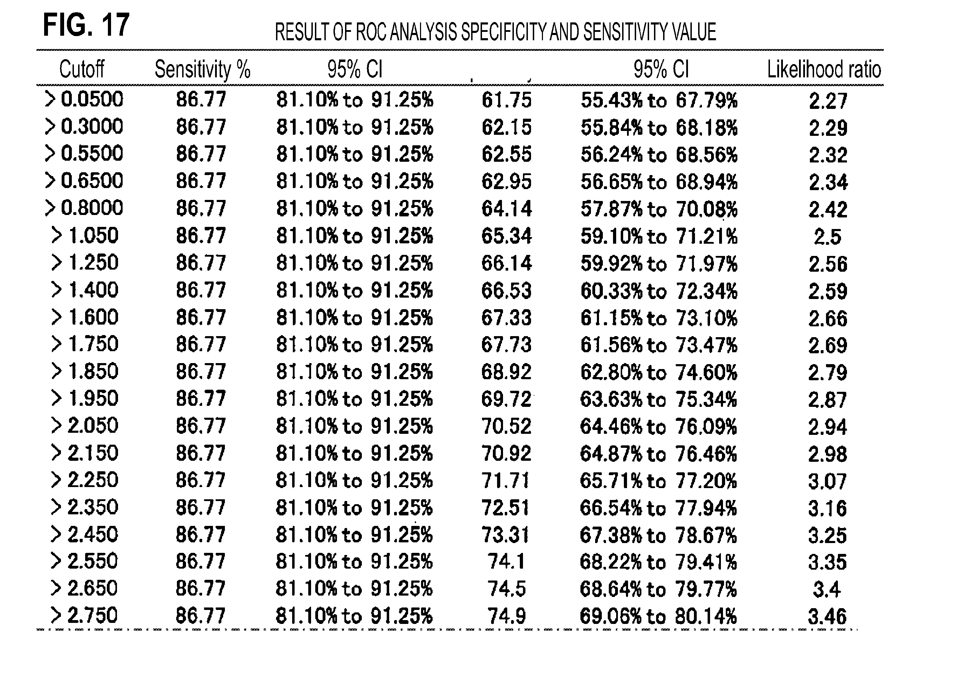

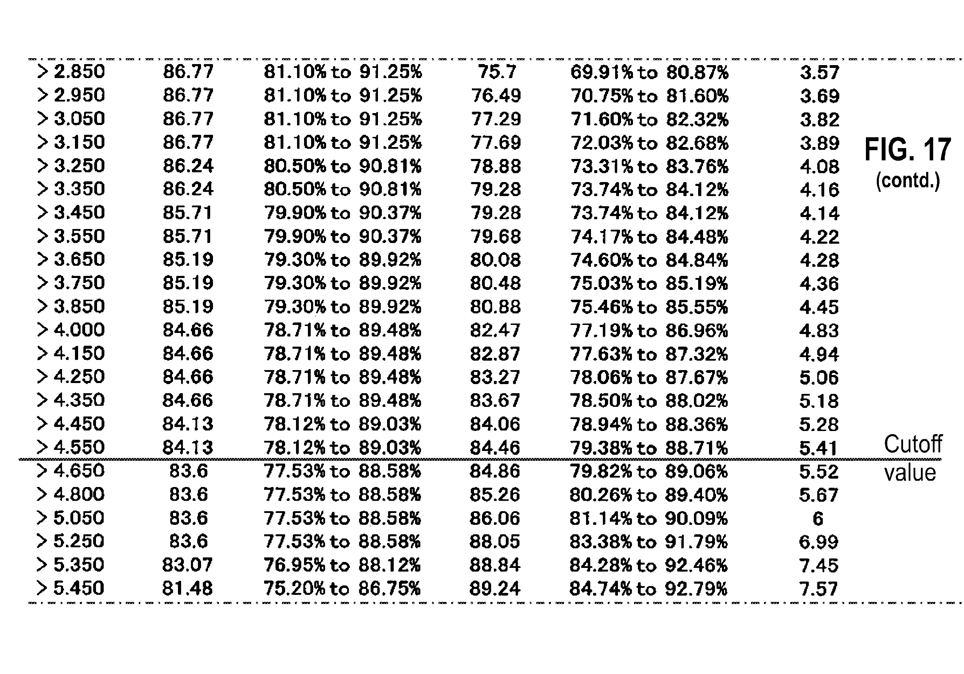

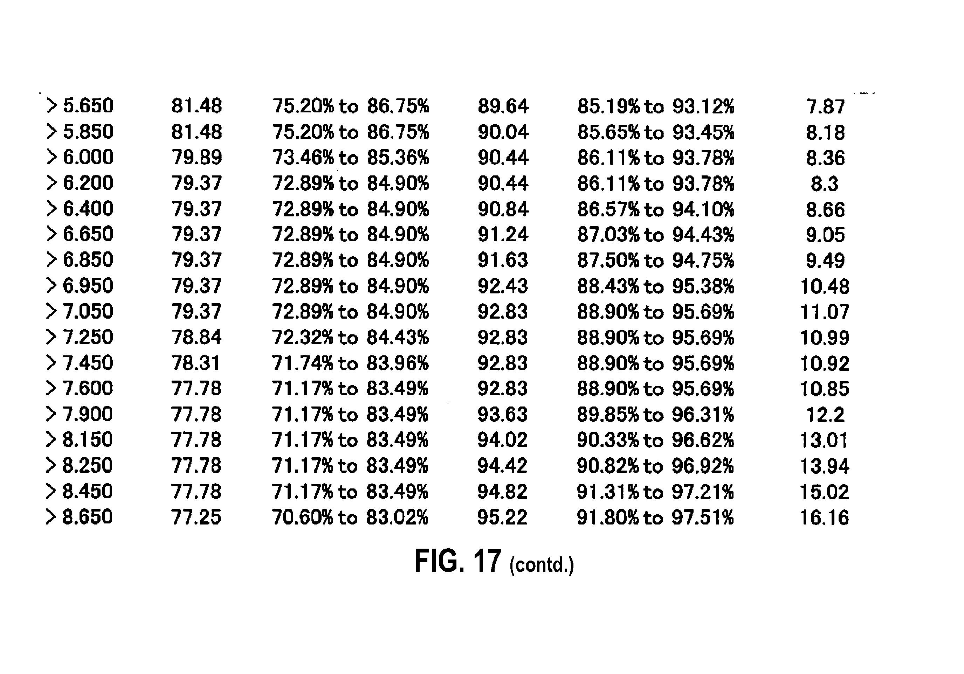

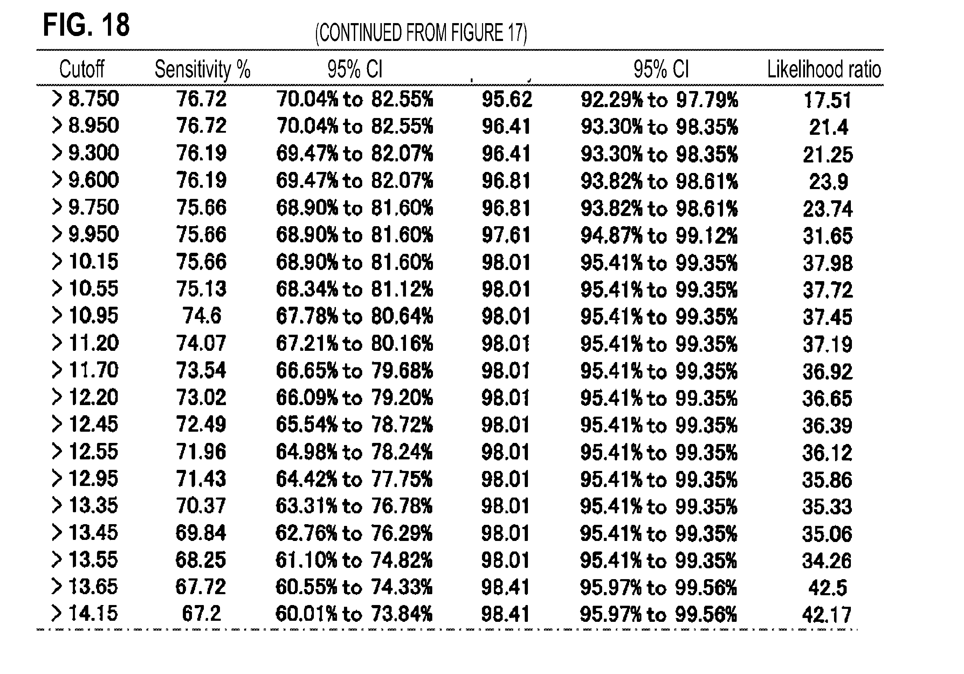

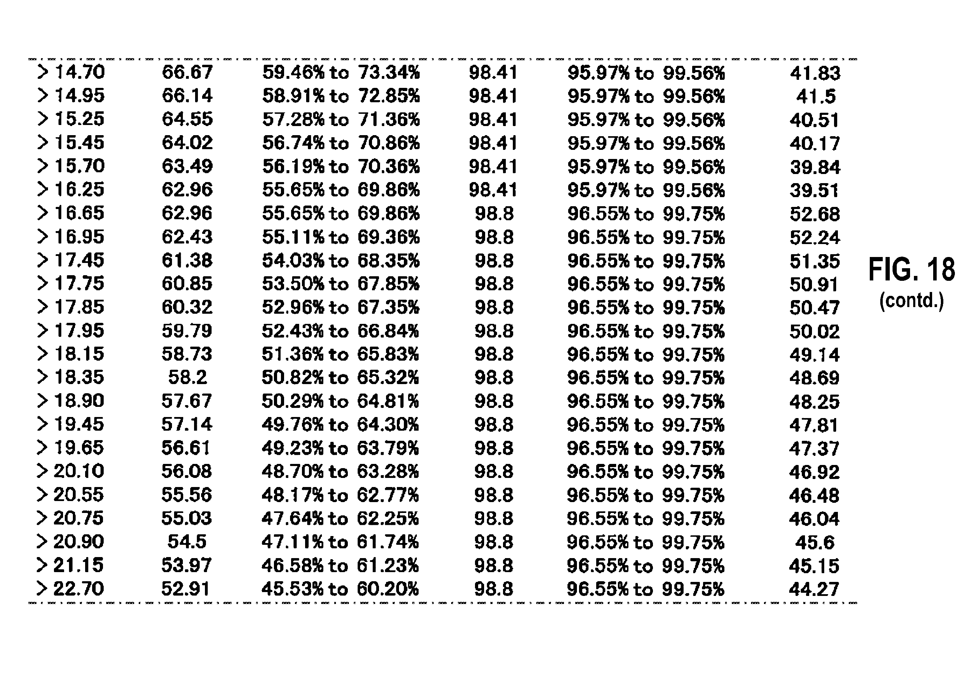

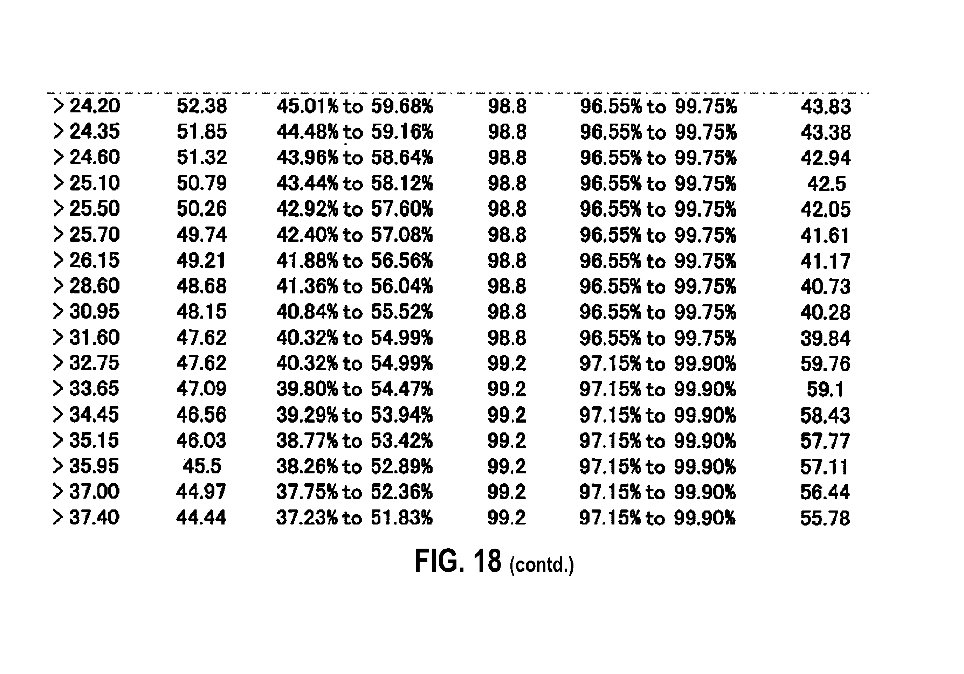

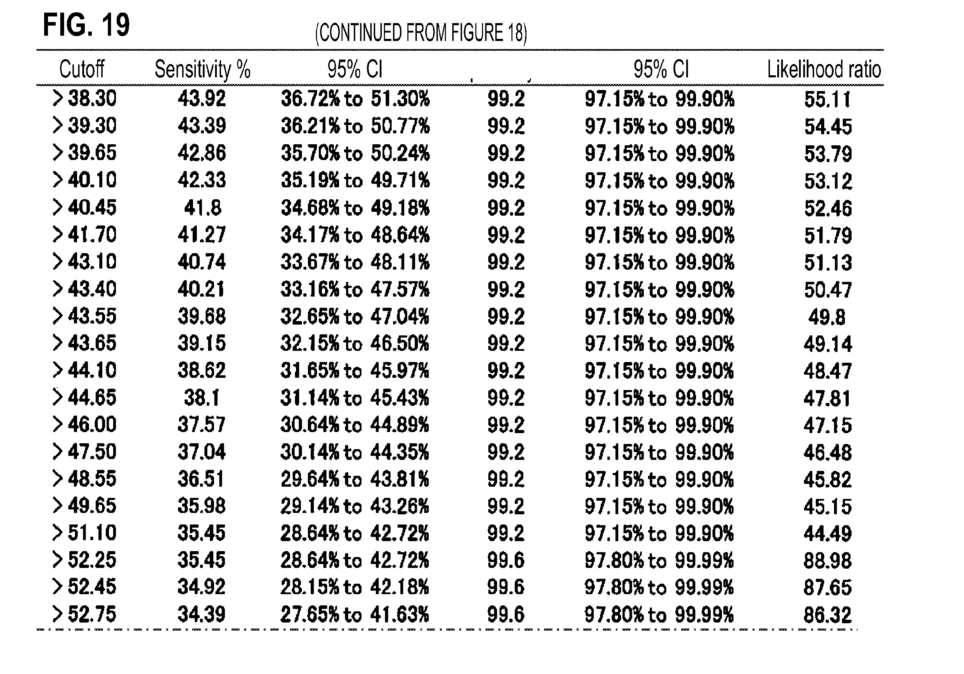

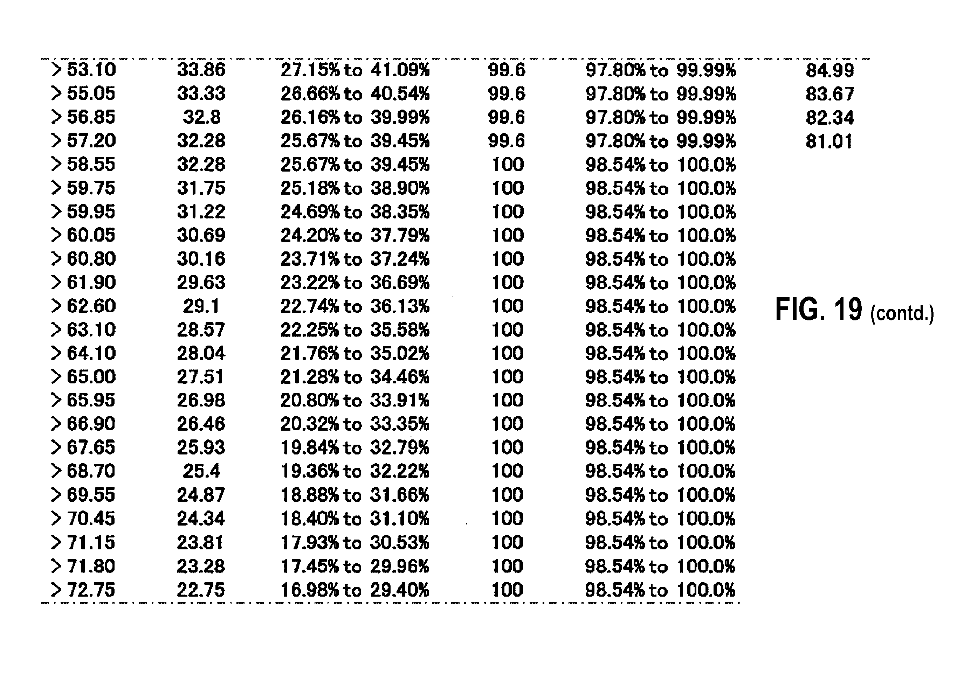

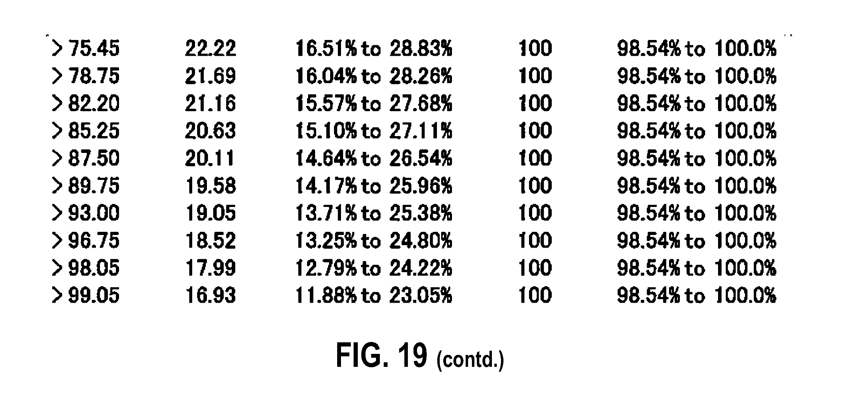

Figure 16 shows an ROC curve prepared using data on normal colorectal mucosa and data on colorectal adenoma. In the graph, the ordinate axis represents the detection sensitivity and the horizontal axis represents 100.0% specificity. The data used for the ROC analysis were also shown in Figures 17 to 19. In the figures, "Cutoff" represents a cutoff value; "Sensitivity", the detection sensitivity (%); "95% CI", 95% confidence interval; and "Specificity", specificity (%). "Likelihood ratio" shows the ratio of likelihood. Figures 17 to 19 are divisions of data whose cutoff values are from 0.05 to 99.05; the likelihood ratios after Cutoff = 57.20 are not determined. From the graph in Figure 16 and Figures 17 to 19, the methylation level = 4.55% at which the sensitivity and specificity had optimal values (shown in boldface numbers and underlines) was considered to be a cutoff value.

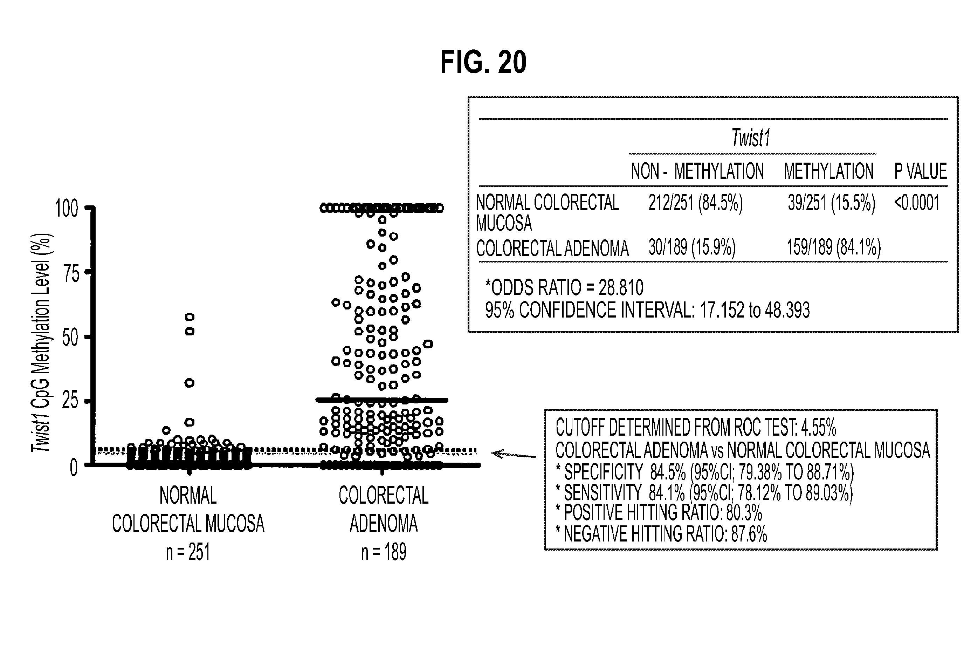

Figure 20 is a graph in which the cutoff value was applied to the graph of the methylation frequency of normal colorectal mucosa and colorectal adenoma. The cutoff value (4.55%) is shown in dotted lines on the graph, and the ratios of methylated/unmethylated DNA in normal colorectal mucosa and colorectal adenoma when the cutoff value is applied are shown in a box at the right top of the graph. When the cutoff value of 4.55% was applied, the percentage of methylated DNA in colorectal adenoma was 84.1% (159/189), while that in normal colorectal mucosa had an extremely low value of 15.5% (39/251). The sensitivity and specificity derived from the value were 84.1% and 84.5%, respectively. The positive hitting ratio was 80.3% (159/198) and the negative hitting ratio was 87.6% (212/242).

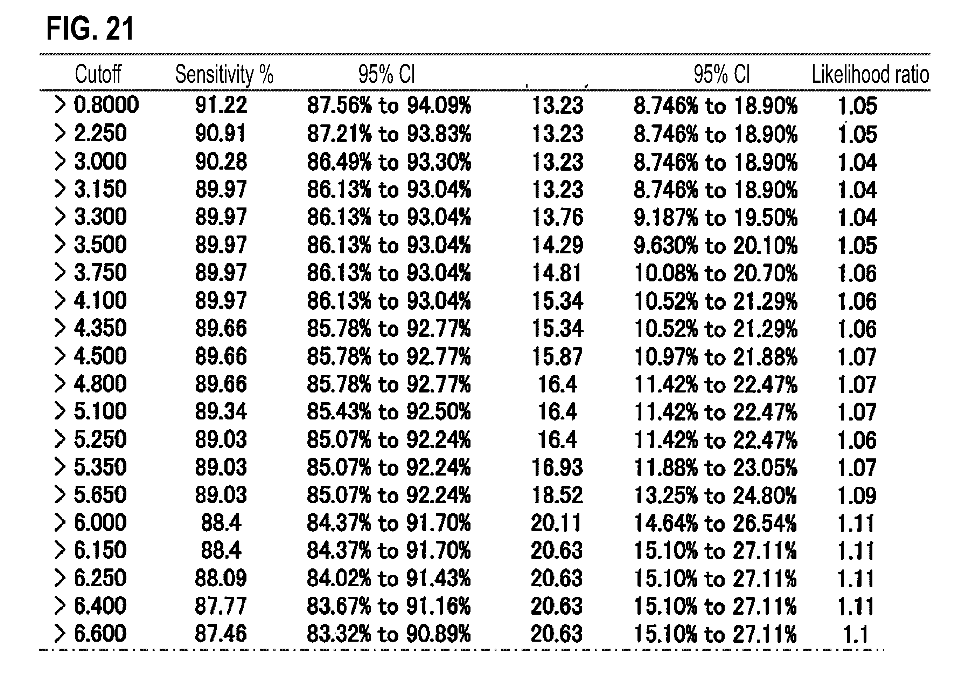

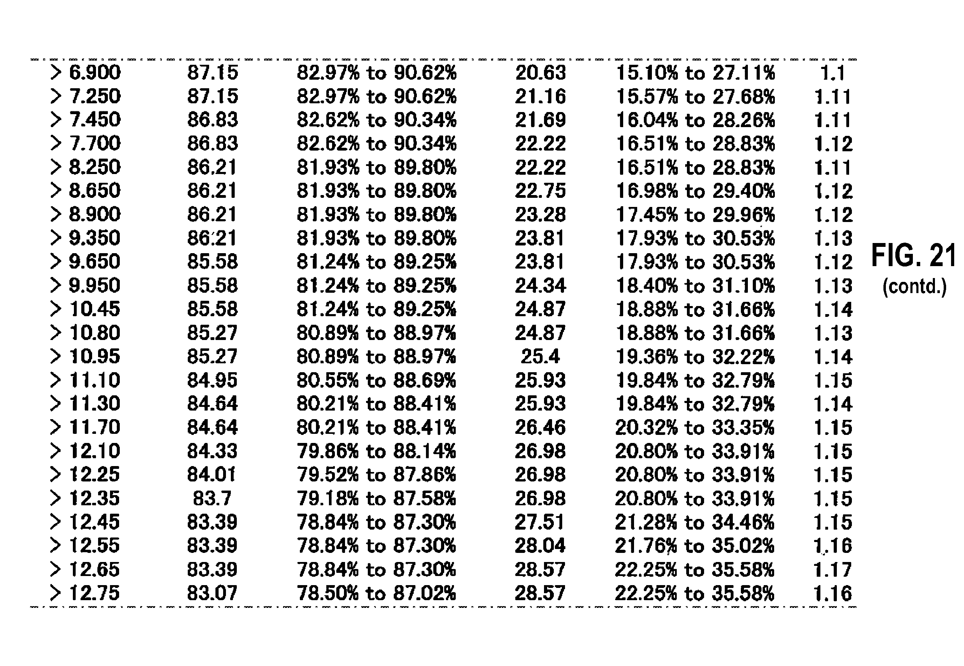

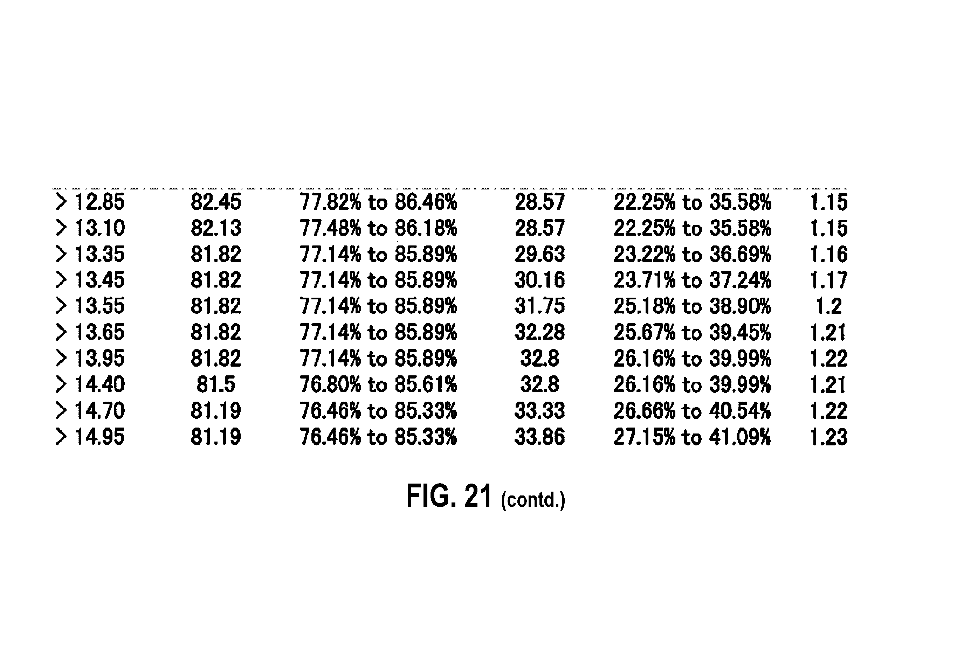

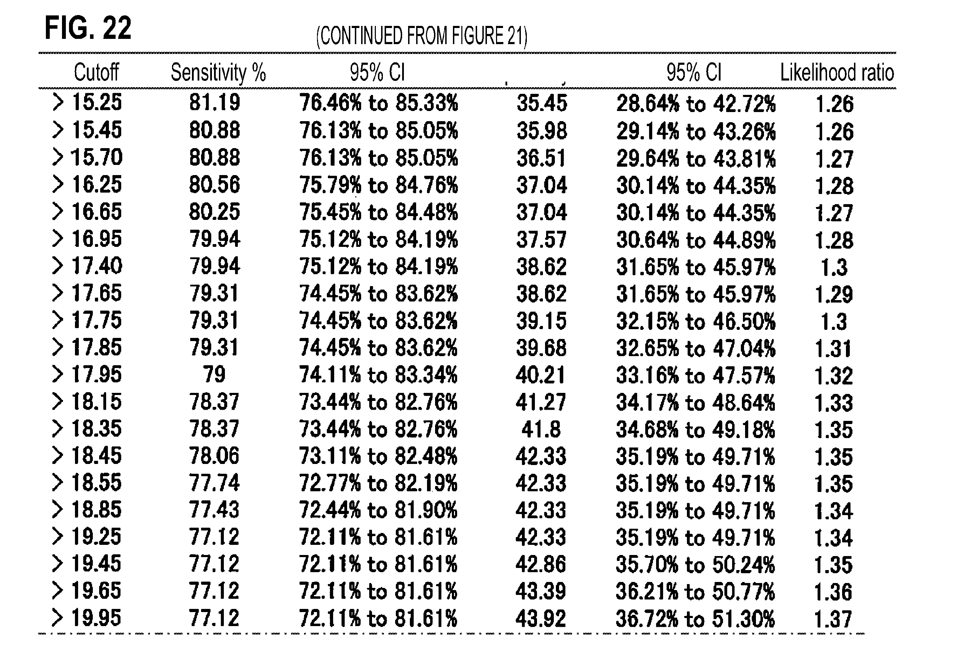

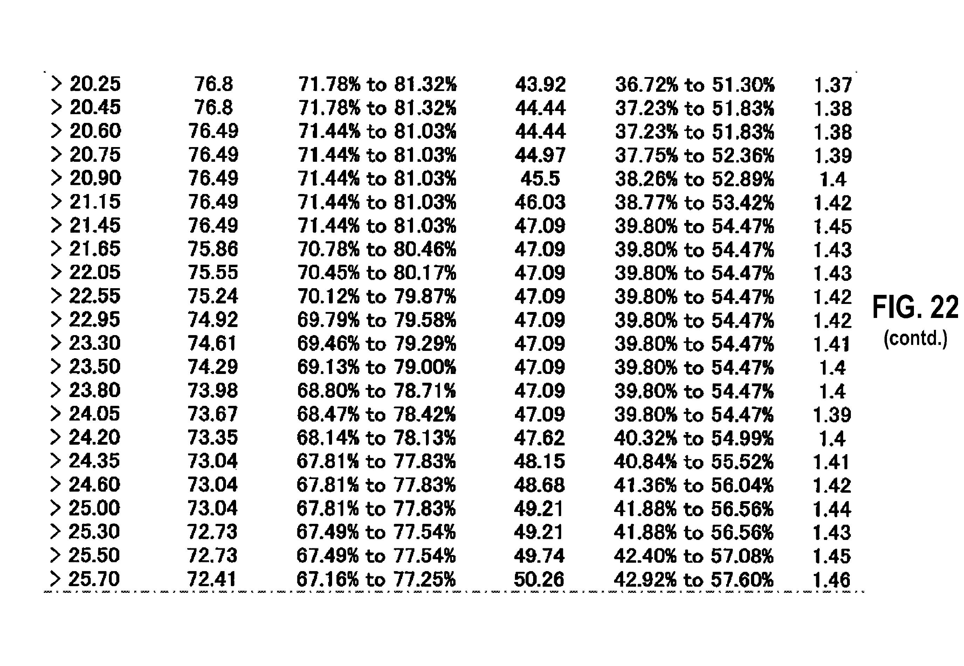

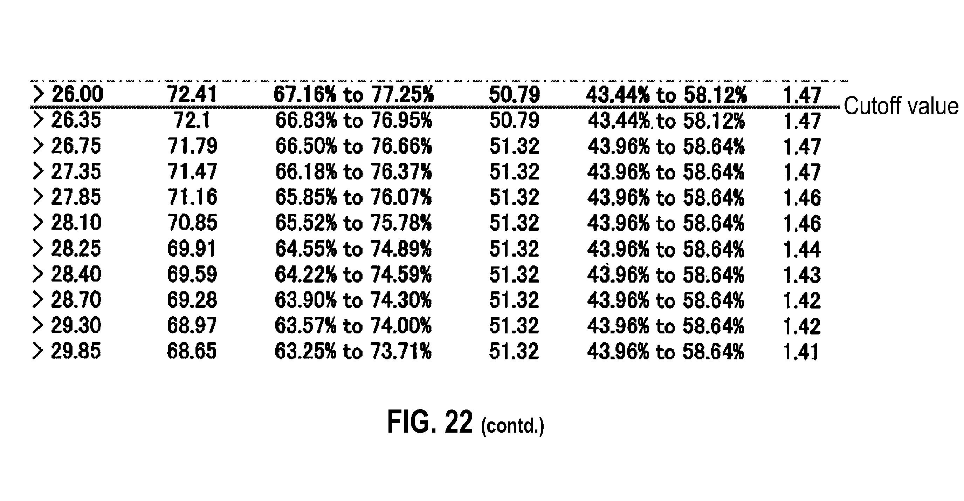

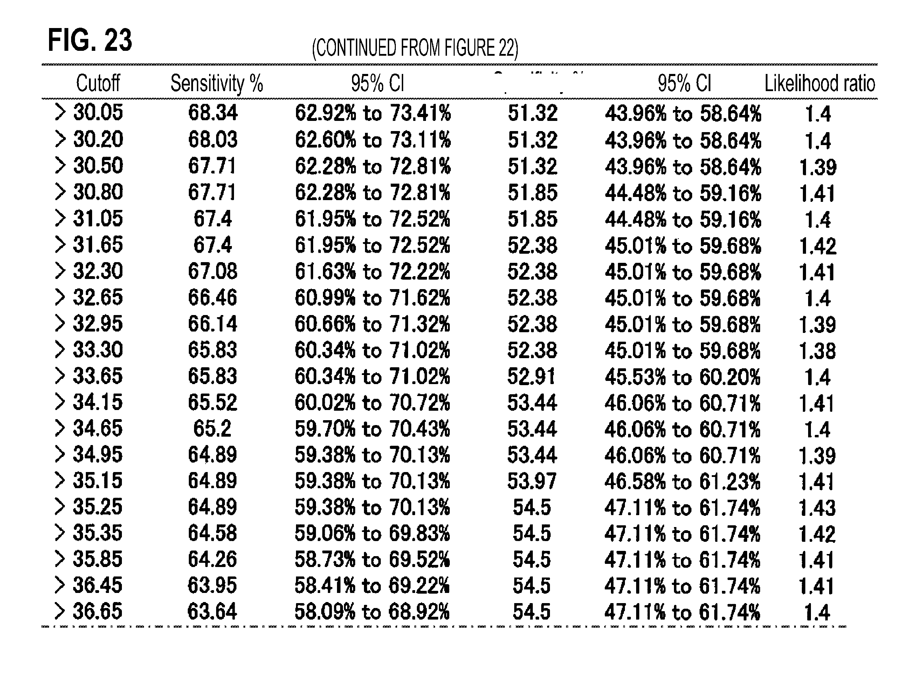

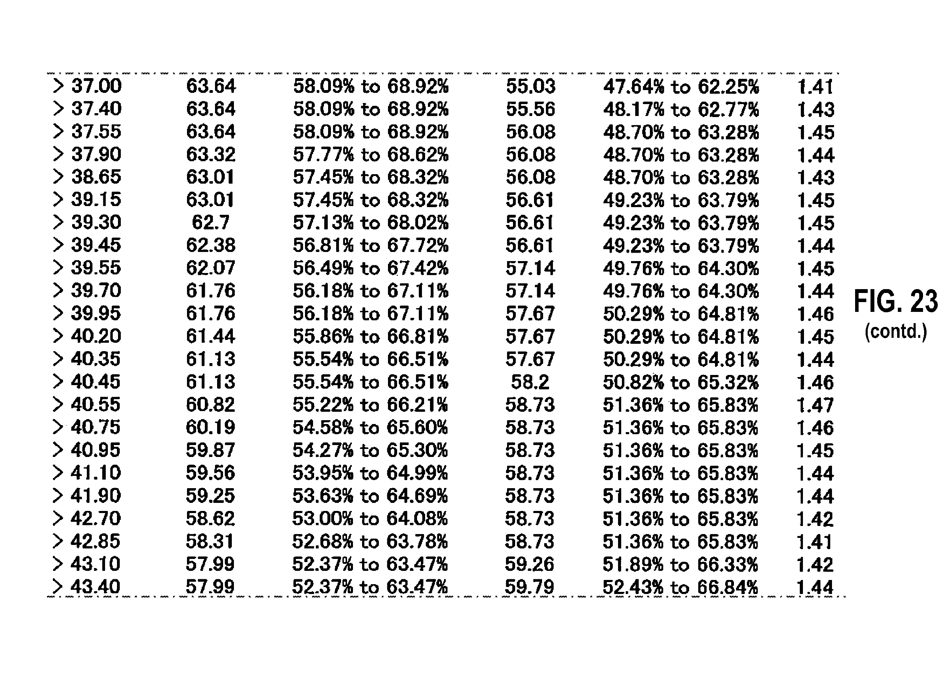

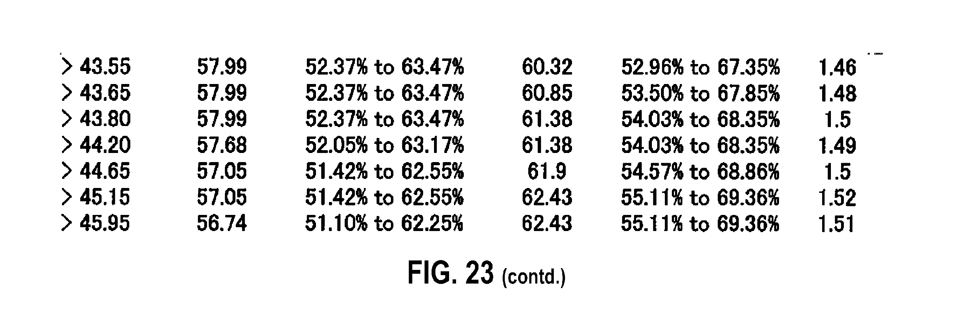

The data obtained in the above Examples 1 and 2 were used to attempt the discrimination between colorectal adenoma and colorectal cancer based on the methylation frequency. Based on the data used in Figure 15, the optimal cutoff value in colorectal adenoma (the methylation frequency is relatively low) and colorectal cancer (the methylation frequency is relatively high) was examined by ROC analysis.
The results of the examination are shown in Figures 21 to 23. In the tables, "Cutoff" represents a cutoff value; "Sensitivity", the detection sensitivity (%); "95% CI", 95% confidence interval; and "Specificity", specificity (%). "Likelihood ratio" shows the ratio of likelihood. Figures 21 to 23 are divisions of data whose cutoff values are from 0.8000 to 45.95. From Figures 21 to 23, the methylation level at which the sensitivity and specificity had optimal values (shown in boldface numbers and underlines in the figures) was considered to be 26.0%. When the cutoff value of 26.0% was applied, colorectal cancer and colorectal adenoma could be differentiated at a detection sensitivity of 72.4% and a specificity of 50.8% and with probabilities of a positive hitting ratio of 71.3% and a negative hitting ratio of 52.2%. This showed that the marker provided by the present invention could be used as a marker capable of detecting not only the presence or absence of tumor but also the degree of progression (benignity/malignancy) thereof.

### Example 3

### (Use of Colorectal Cancer Cell Line)

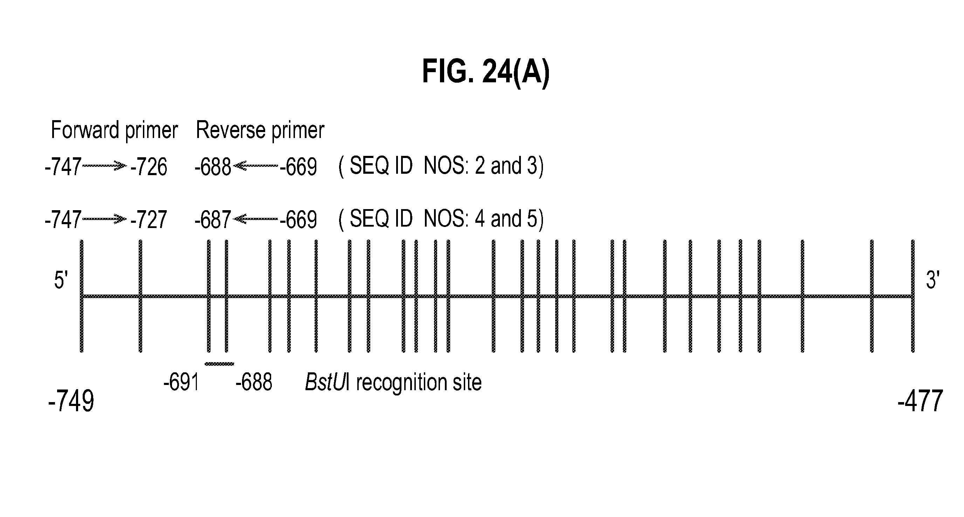

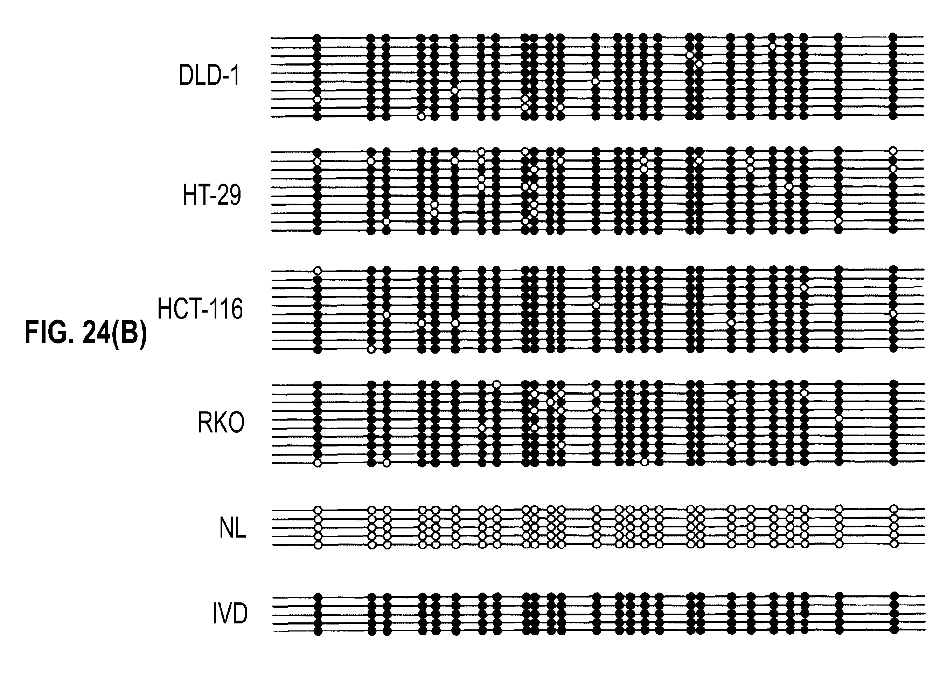

Colorectal cancer cell lines were each used to examine the methylation of CpG sequences within the region between positions -477 and -747 of TWIST1 gene, specifically the methylation of CGCG at positions 57 to 60 and CpG at other places in the base sequence shown in SEQ ID NO: 1. DLD-1, HT-29, HCT-116, and RKO were used as colorectal cancer cell lines, and NL as peripheral blood lymphocyte-derived cells was employed as a methylation negative control and IVD as methylation transferase-treated DNA was employed as a methylation positive control. The methylation patterns of 10 clones (the methylation pattern of each of 10 cells) for the colorectal cancer cell lines (DLD-1, HT-29, HCT-116, and RKO) and the methylation patterns of 5 clones each of the methylation positive control (IVD) and the methylation negative control (NL) were examined. Figure 24 is a chart showing the distribution of CpG methylation between positions -477 and -747 of TWIST1 gene. The black circle represents the presence of CpG methylation and the white circle represents the absence of methylation. The methylation pattern was analyzed by a COBRA method using BstUI or a bisulfite sequencing method.

### (Results)

The methylation pattern of CGCG at positions 57 to 60 in the base sequence (the region between positions -691 to -688 in the gene) was confirmed to correlate well with the methylation pattern of CpG in the region between positions -477 and -747 of the gene. Thus, it was shown that if methylation was present in CGCG at positions 57 to 60, methylation was also observed in other sites.

### Comparative Example 3

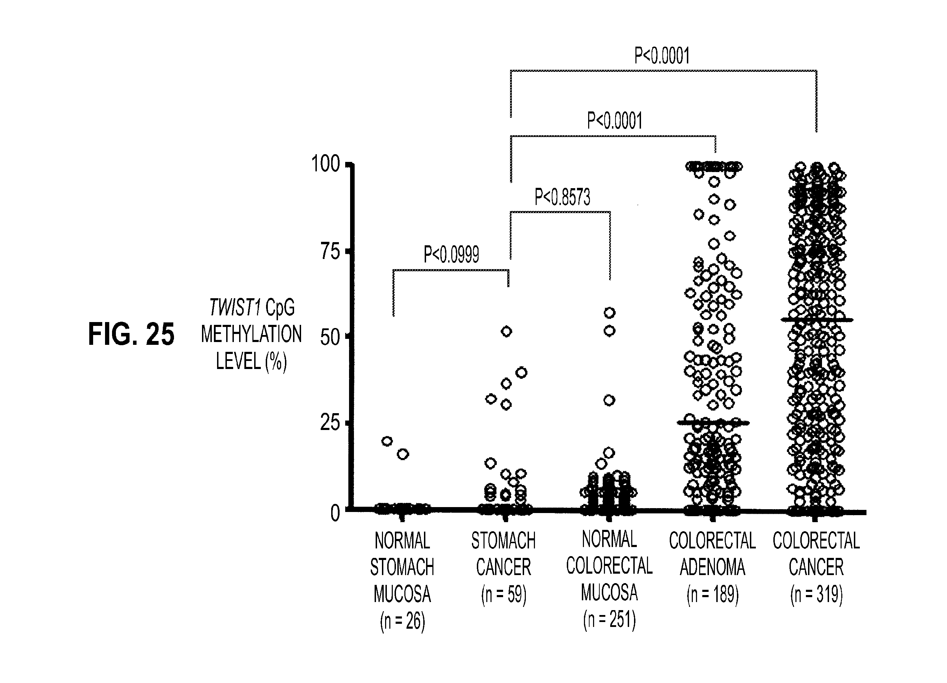

Using stomach cancer cells and normal stomach mucosa, the CpG methylation levels (%) of TWIST1 therein were compared as described in Example 1. As a result, no preponderance was observed. The results are shown in Figure 25.

### Example 4

### (Detection of Cell from Clinical Specimen)

A stool collected from a subject was filtered using a mesh. Then, to select cells expressing EP-CAM known to be widely expressed on the basolateral surface of epithelial cells, anti-EP-CAM antibody was added thereto. After stirring the resultant, an iron bead antibody was used to drive a secondary antibody response. After again stirring, target cells were adsorbed to a column through a magnet to recover the cells. The methylation pattern of TWIST1 gene was analyzed according to the method of Example 1 except for performing the extraction of DNA from the recovered cells using QIAamp Stool DNA Isolation Kit (from Qiagen).

### Industrial Applicability

The use of the method for determining the presence or absence of colorectal tumor according to the present invention enables the development of a kit and the like for sensitively detecting colorectal tumor such as colorectal cancer and colorectal adenoma with high specificity. This provides an extremely useful tool for the detection of tumor with which early detection is most important, particularly cancer.

## Claims

1. A method for determining a presence or absence of colorectal tumor, comprising measuring a degree of methylation of one or more CpG sequence (s) in the region between positions -477 and -747 of TWIST1 gene in the genome sequence of a test cell.

2. The determination method according to claim 1, wherein when it is determined that there is a colorectal tumor, the colorectal tumor is further determined to be a colorectal cancer or colorectal adenoma.

3. The determination method according to claim 1 or 2, wherein the one or more CpG sequence (s) in the region between positions -477 and -747 of TWIST1 gene is the CGCG sequence located at positions 57 to 60 in the nucleotide sequence represented by SEQ ID NO: 1.

4. The determination method according to claim 3, wherein the degree of methylation is measured by a COBRA (Combined Bisulfite Restriction Analysis) method and the CGCG sequence located at positions 57 to 60 in the nucleotide sequence represented by SEQ ID NO: 1 is amplified using a combination of 5'-TGTGTAGAAGTTGTTGTTATT-3' (SEQ ID NO: 4) and 5'-CRAAAAAAACTATCCTAAC-3' (SEQ ID NO: 5) as a primer set in an amplification step.

5. The determination method according to any one of claims 1 to 4, wherein the test cell is a cell derived from tissue suspected of harboring colorectal cancer or a precancerous lesion, a cell contained in feces, a cell contained in a colorectal wash, a cell contained in blood, or a cell contained in serum.

6. A colorectal tumor determination kit for measuring a degree of methylation of one or more CpG sequences in a region between positions -477 and -747 of TWIST1 gene in the genome sequence of a test cell, comprising a reagent for extracting genomic DNA, a reagent for converting unmethylated cytosine to uracil, a primer set for amplifying one or more CpG sequences in the region between positions -477 and -747 of TWIST1 gene in the genomic DNA, and a reagent for analyzing a methylation pattern.

7. The colorectal tumor determination kit according to claim 6, wherein the primer set is a primer pair consisting of the oligonucleotides represented by SEQ ID NO: 4 and SEQ ID NO: 5.
